# DEMANDE DE BREVET EUROPEEN

(11) **EP 3 686 378 A1**
(43) Date de publication de la demande: **29.07.2020**
(21) Numéro de dépôt: 19153558.2
(22) Date de dépôt: 24.01.2019
(51) Int. Cl.: E05B 1/00, A61L 2/18, E05B 47/00

(54) **DISPOSITIF DE NETTOYAGE AUTOMATIQUE D'UNE POIGNÉE**

(71) Demandeur: Horvath, Alex, 1005 Lausanne (CH); van Sprolant, Valentin, 74380 Bonne (FR); Barilla, Giovanni, 1206 Genève (CH)
(72) Inventeur: Horvath, Alex, 1005 Lausanne (CH); van Sprolant, Valentin, 74380 Bonne (FR); Barilla, Giovanni, 1206 Genève (CH)
(74) Mandataire: Reuteler, Raymond Werner

(57) **Abrégé**

L'invention se rapporte à un dispositif de nettoyage d'une poignée (1) comprenant une poignée (2) comprenant une surface de préhension (3) à désinfecter, un organe d'application (10) pour appliquer un liquide nettoyant sur la surface de préhension (3), des moyens d'alimentation (12) en liquide nettoyant comprenant au moins un réservoir (11) pour recevoir et distribuer du liquide nettoyant à l'organe d'application. L'organe d'application (10) comprend au moins une réserve tampon (24) agencée au contact de la surface de préhension (3). Le dispositif comprend une unité d'actionnement (16) de l'organe d'application comprenant des moyens d'entraînement configurés pour déplacer la réserve tampon (24) et/ou la surface de préhension (3) relativement l'une par rapport à l'autre de façon à appliquer du liquide nettoyant sur sensiblement toute la surface de préhension lors de ce déplacement.

## Description

### DOMAINE TECHNIQUE DE L'INVENTION

La présente invention concerne un dispositif de nettoyage automatique d'une poignée par exemple une poignée d'ouverture d'un ouvrant telle qu'une porte, une fenêtre, porte-fenêtre ou une poignée d'appui ou encore une rambarde ou une rampe.

### ÉTAT DE LA TECHNIQUE ANTERIEURE

Dans certains lieux publics à forte fréquentation et/ou sensibles, comme les toilettes, les hôpitaux, les blocs opératoires, les hôtels, restaurants, les commerces, les bureaux, etc., il est important de maintenir une bonne hygiène sur les principales surfaces de contact pour éviter la transmission de maladies. Les poignées présentent des surfaces sur lesquelles se concentrent les bactéries, virus ou autres microorganismes. Un dépôt gras s'accumule aussi sur ces surfaces. Il est donc important et recommandé de les nettoyer régulièrement avec un produit nettoyant et de préférence, ayant des propriétés désinfectantes.

Ainsi, le nettoyage des surfaces comme les poignées est souvent réalisé de manière irrégulière et incomplète. Or la transmission de microorganismes (virus, bactéries, champignons, etc.) peut se faire facilement et rapidement par simple touché d'une surface infectée. Il est donc difficile d'éliminer le risque de transmission par les méthodes traditionnelles de nettoyage.

Le brevet US7360674 se rapporte à un distributeur d'un produit germicide à partir d'une cartouche pour la désinfection d'une poignée de porte. Le produit est vaporisé sous forme d'aérosol sur la surface de la poignée sous le contrôle d'une vanne. Toutefois il est difficile avec un tel système d'assurer pas une couverture homogène et complète de la surface de la poignée par le produit ainsi vaporisé. Un excès de produit peut conduire à un ruissèlement ou fuite de liquide sur le sol. En fonction de la température et des courants d'air, l'aérosol peut être transporté dans la pièce et être inhalé accidentellement par les personnes ou s'introduire dans leurs yeux ; ce qui peut conduire à des désagréments (mauvais goût, irritation, rougeurs, etc.). La solution hydro-alcoolique risque aussi de détériorer la porte et son environnement. L'alcool peut dégrader la peinture de la porte après une exposition prolongée.

Le brevet EP3118395 se rapporte à un dispositif de désinfection d'une poignée d'actionnement d'une porte présentant un récipient contenant un agent de désinfection fluide qui parvient sur la surface de la poignée au travers de plusieurs ouvertures sous forme d'incisions, réalisées dans une surface extérieure flexible, s'ouvrant sous l'effet de la pression exercée lors de l'actionnement de la poignée. Un premier inconvénient provient de ce que la distribution du liquide se fait dans la main de l'utilisateur, ce qui n'est pas toujours souhaitable. Un autre inconvénient est que le dosage du fluide désinfectant est fonction de la pression d'actionnement sur la poignée. En particulier, si la pression sur la surface de la poignée est insuffisante à l'ouverture des incisions, le passage du fluide ne se fait pas ou insuffisamment et la poignée est donc mal désinfectée.

La demande de brevet DE102010011309A1 concerne une poignée désinfectée au moyen de rayons ultraviolets ou une lumière au plasma. Cette méthode requière une source d'énergie permanente. Son efficacité de désinfection dépend largement de l'arrangement des sources lumineuses, de leur puissance et du temps d'exposition. Une méthode similaire est décrite dans la demande US20100140499A1 et le brevet US7989779B1.

La demande de brevet US20120176241 A1 se rapporte à un dispositif de stérilisation d'une poignée de porte utilisant un bouclier mobile qui enveloppe la poignée lors de la désinfection pour la désinfecter comme par ultraviolet et se déplace pour libérer la poignée lorsque la présence d'un utilisateur est détectée. Le bouclier rend le dispositif encombrant et nécessite un mécanisme de déplacement important et qui consomme beaucoup d'énergie électrique. Il nécessite aussi une détection de proximité pour libérer la poignée ; ce qui peut poser des problèmes de fonctionnement et d'un temps d'attente avant de pouvoir se saisir de la poignée ainsi libérée.

La demande de brevet US20140137369A1 se rapporte à une poignée de porte autonettoyante comprenant une poignée annulaire montée sur un bâti qui se déplace dans un réservoir contenant une solution de désinfection ; le réservoir comprenant un joint qui racle la surface de la poignée pour enlever l'excès de solution. Une source de rayons ultraviolets est disposée sur le bâti pour exposer la poignée à ce rayonnement pendant l'activation de la rotation de la poignée. Un tel dispositif a pour inconvénients de nécessiter une poignée peu fonctionnelle, utilisant un mécanisme d'entraînement compliqué et encombrant et dont l'ergonomie pour la préhension n'est pas satisfaisante. Le remplissage du réservoir est aussi compliqué à réaliser.

Il existe donc un besoin pour proposer une solution satisfaisante pour le nettoyage d'une poignée, dont les fonctionnalités et l'ergonomie sont conservées, qui soit efficace, simple et préférablement autonome dans son mode de fonctionnement d'un point de vue énergétique.

### EXPOSE DE L'INVENTION

L'invention se rapporte ainsi à un dispositif de nettoyage d'une poignée comprenant :
au moins une poignée comprenant :
- une surface de préhension à nettoyer, de préférence faisant partie d'une paroi de forme tubulaire ou partiellement tubulaire,
- un organe d'application pour appliquer un liquide nettoyant sur la surface de préhension,
- des moyens d'alimentation en liquide nettoyant comprenant au moins un réservoir pour recevoir et distribuer du liquide nettoyant à l'organe d'application,
caractérisé en ce que l'organe d'application comprend au moins une réserve tampon agencée au contact de la surface de préhension et en ce que le dispositif comprend une unité d'actionnement de l'organe d'application comprenant des moyens d'entraînement configurés pour déplacer l'organe d'application relativement à la surface de préhension et/ou pour déplacer la surface de préhension relativement à l'organe d'application, de façon à appliquer du liquide nettoyant depuis la réserve tampon sur la surface de préhension lors de ce déplacement relatif.

Ainsi, un aspect de l'invention se base sur le principe de la mobilité relative d'un organe de nettoyage sur la surface contaminée et contenant un volume de liquide réduit, de préférence prédosé en fonction de la surface à nettoyer. Ainsi, le dispositif est efficace, moins encombrant et ses besoins en énergie pour le faire fonctionner sont plus faibles que les dispositifs connus. La réserve tampon est préférablement configurée de façon à contenir un volume de liquide nettoyant déterminé pour nettoyer la totalité de la surface de préhension lors du déplacement relatif. De ce fait, le nettoyage est complet et reproductible à chaque cycle. Le rechargement de la réserve tampon par l'activation des moyens d'alimentation en liquide peut aussi se faire en temps masqué, et non pas au moment du nettoyage lors du déplacement relatif de l'organe d'application contre la surface à traiter. Il est ainsi plus facile de contrôler la dose de liquide nécessaire au nettoyage de la surface. Il en résulte un moindre risque de défaut d'alimentation en liquide lors de l'étape de nettoyage. Enfin, cela rend possible par des moyens de collecte (décrits plus loin) l'utilisation de l'énergie produite par l'actionnement mécanique de la poignée comme lors de l'ouverture de l'ouvrant.

La surface de préhension n'est pas nécessairement tubulaire et peut être partiellement tubulaire au sens où elle peut former une surface en partie ouverte longitudinalement comme une surface en forme transversale hélicoïdale, en U ou en C ou autre.

Selon une autre configuration possible, la surface de préhension pourrait être par exemple formée d'une série de plusieurs tubes de petits diamètres arrangés individuellement en rotation libre selon leur propre axe individuel et arrangés ensemble dans une disposition circulaire autour d'un axe médian longitudinal de la poignée formant ainsi un ensemble tubulaire ou semi-tubulaire de plus grand diamètre.

Le terme « nettoyage » se rapporte essentiellement à une opération de désinfection (c'est-à-dire de destruction de microorganismes ou germes) et possiblement de dégraissage. Le terme « liquide nettoyant » se rapporte essentiellement à un liquide ayant des propriétés désinfectantes et possiblement dégraissantes. De préférence le liquide est constituée ou contient une solution hydro-alcoolique et/ou une solution contenant des agents tensio-actifs ou savonneux capable d'éliminer ou dissoudre les corps gras.

De préférence, les moyens d'alimentation en liquide comprennent une pompe qui peut être actionnée de manière contrôlée électroniquement ou mécaniquement par actionnement de la poignée. La pompe peut être une pompe péristaltique ou une pompe à piston ou au d'un autre type. Dans un arrangement possible, la pompe est configurée pour alimenter la réserve tampon sous l'effet d'une action mécanique appliquée sur la pompe par au moins un élément d'actionnement de la poignée associé au mouvement de la poignée en rotation lors de l'actionnement d'un mécanisme d'ouverture et/ou de fermeture de l'ouvrant.

Selon un aspect préférentiel de l'invention, la réserve tampon comprend un tampon applicateur en contact avec la surface de préhension ayant une structure permettant de stocker le liquide dans une structure poreuse ou d'interstices de rétention du liquide tel que par effet de capillarité. Une telle configuration de la réserve tampon a pour avantage de pouvoir mieux réguler l'application et l'étalement du liquide sur la surface, tout en minimisant avantageusement le risque de fuites. Il a aussi pour avantage de mettre à disposition un volume de liquide dimensionné pour couvrir toute la surface de préhension lors d'un seul cycle de déplacement relatif (surface de préhension/organe d'application) lors du nettoyage.

Le matériau du tampon applicateur peut être avantageusement compressible. La compressibilité assure aussi une meilleure régularité de l'application et réduit le risque de spots non-couverts par le liquide. Le matériau peut être : une éponge ou une mousse, une brosse de fils, une épaisseur ou un enroulement de fibres, une structure en nid d'abeille, des billes en polymère ou tout matériau muni d'une multitude de cavités ou interstices de faibles volumes formant un réseau de régulation du flux de sortie et/ou d'étalement du liquide. La capacité en liquide de la réserve tampon dépend de la surface de préhension à traiter mais peut être par exemple comprise entre 0.05 à 3 ml, de préférence entre 0.1 et 1 ml.

La dimension transversale du tampon applicateur peut être prévue pour que le tampon applicateur couvre toute la surface à traiter lors du déplacement relatif. Le nettoyage peut ainsi être obtenu selon les cas par un déplacement unidirectionnel ou d'un seul passage ou par un déplacement bidirectionnel ou selon un aller-retour de l'organe d'application sur la surface de préhension ou inversement. Dans tous les cas, le déplacement est de préférence le plus court possible et ne nécessite pas de mouvements ou de trajets ou trajectoires complexes. De préférence, le tampon applicateur est dimensionné à la largeur (c-à-dire correspondant à une dimension un peu supérieure à la largeur d'une main) ou au diamètre de la surface de préhension.

Selon un aspect de l'invention que le dispositif comprend un collecteur de distribution de liquide nettoyant comprenant au moins une entrée de liquide en communication avec le réservoir de liquide et une pluralité de sorties de liquide distribuée en regard de la réserve tampon, de préférence du tampon applicateur de l'organe d'application. Ainsi, un arrangement avec plusieurs sorties de liquide permet de distribuer mieux et plus rapidement le liquide au travers du tampon applicateur notamment sur toute sa largeur. Cet arrangement augmente l'efficacité de recouvrement de la surface traitée, évite les zones surchargées en liquide et réduit aussi le volume total de liquide nécessaire pour un cycle de nettoyage.

Selon un mode possible, la poignée comprend une embase fixe sur laquelle est fixé l'organe d'application et sur laquelle la paroi de la surface de préhension est montée mobile selon au moins une direction en rotation autour de l'axe longitudinale de la poignée ; les moyens d'entraînement étant arrangés pour entraîner la paroi tubulaire en rotation contre l'organe d'application pour effectuer l'application d'une couche de liquide nettoyant sur la surface de préhension lors du déplacement selon ladite direction en rotation de la surface de préhension. Ainsi, l'application du liquide sur la surface à nettoyer se fait par au moins une rotation de la surface de préhension elle-même ; le tampon applicateur restant statique vis-à-vis de l'embase de la poignée ou étant mobile selon au moins une direction diamétrale ou radiale vis-à-vis de la surface de préhension. Cet arrangement possède l'avantage d'être de construction simple et de limiter les déplacements. Une rotation angulaire de l'ordre de 360 degrés est suffisante pour réaliser l'étalement d'une couche de liquide nettoyant contre la surface.

Selon un mode de réalisation avantageux, la surface de préhension de la paroi tubulaire mobile a une forme transversale sensiblement hélicoïdale et comprend une extension de couverture en porte-à-faux configurée pour recouvrir l'organe d'application en position de repos du dispositif ; ladite extension de couverture formant un logement pour ledit organe muni d'une ouverture s'étendant à la fois axialement et circonférentiellement pour permettre le passage de l'organe d'application lors du déplacement en rotation de ladite surface de préhension. Dans la configuration de repos entre deux cycles de nettoyage, l'organe d'application est ainsi recouvert par la surface de préhension évitant ainsi d'être lui-même contaminé lorsque l'utilisateur touche la poignée. Ainsi, l'unité d'actionnement est configurée pour entraîner la surface de préhension selon une première rotation depuis logement en direction de ladite extension de couverture pour effectuer l'application d'une couche liquide sur la surface de préhension puis selon une seconde rotation dans la direction opposée jusqu'au rangement de l'organe d'application dans le logement.

Dans un premier cas possible, une double rotation de la surface de préhension à +360 et -360 degrés peut être prévue pour permettre à toute la surface d'être recouverte, y compris l'extension de couverture et permettre à l'organe d'application d'être disposé ensuite dans le logement en position de repos. Plus particulièrement, l'unité d'actionnement est configurée pour entraîner la surface de préhension en première rotation d'environ 360° selon une première direction depuis une position de repos de l'organe d'application dans ledit logement de l'extension de couverture jusqu'à une position de fin de course de l'organe d'application sur l'extension de couverture suivie d'une seconde rotation d'environ 360° dans la direction opposée jusqu'au retour de l'organe d'application dans le logement de l'extension de couverture de la surface de préhension. Ainsi, la surface de préhension est commandée dans ce déplacement en aller-retour en rotation pour être nettoyée entièrement et assurer ensuite le rangement de l'organe de nettoyage à l'écart de la surface destinée à être touchée par l'utilisateur qui empoigne la poignée.

Selon une variante possible, le déplacement de la surface de préhension comprend une première rotation supérieure à +360°, par exemple de +380-400° pour permettre à la surface de préhension d'être recouverte sur toute sa circonférence par le liquide et permettre ainsi à la l'organe d'application de passer au-delà de l'extension de couverture et s'arrêter à côté du logement (l'organe d'application faisant un « saut » de la zone haute à la zone basse de la surface de préhension) puis une seconde rotation en sens inverse pour disposer l'organe d'application dans le logement sous l'extension de couverture.

De préférence, l'organe d'application est monté sur l'embase de manière à pouvoir se déplacer radialement ou diamétralement vis-à-vis de la surface de préhension à l'encontre d'au moins un moyen de retour élastique lors de la rotation de ladite surface. De préférence, l'organe d'application est monté le long de deux tiges de guidage placées sur les deux côtés de la surface et en appui contre un ressort coulissant dans chaque tige. Le moyen de rappel élastique permet d'assurer le contact entre l'organe de d'application et la surface de préhension lors de la rotation de la surface de préhension. Ainsi, au fur et à mesure que l'organe d'application se déplace sur la surface hélicoïdale dont le diamètre varie progressivement et continûment, le moyen de rappel élastique le long des tiges de guidage assure le rattrapage de jeu de l'organe vis-à-vis de la surface de préhension.

La paroi tubulaire de préhension de la poignée peut être montée par ses deux extrémités à l'embase au moyen de roulements et/ou bagues de glissement pour réduire les frottements et l'usure pour une utilisation un grand nombre de cycles.

Dans un mode d'actionnement électrique possible du dispositif, les moyens d'entraînement peuvent comprendre une partie de transmission reliée d'une part à un moteur électrique et d'autre part à la surface de préhension. Ces composants peuvent être logés dans le volume interne de la poignée, par exemple sur un noyau de l'embase s'étendant le long de l'axe central de la paroi de la surface de préhension ou sur une autre partie de la poignée.

La partie de transmission peut être du type à engrenage mécanique ou à engrenage sans contact du type électromagnétique ou à vis sans fin. Si nécessaire, la partie de transmission peut être reliée au moteur par un réducteur. Le rapport de réduction sera adapté pour obtenir un temps d'actionnement des moyens d'entraînement aussi court que possible, par exemple, de l'ordre d'une ou deux secondes. Dans un exemple possible, l'engrenage peut comprendre par exemple un premier pignon relié d'une part au moteur et d'autre part à une bague munie d'une surface annulaire dentée interne et solidarisée sur la face interne de la paroi tubulaire de préhension. Un tel agencement a pour avantage d'être peu encombrant tout en produisant un couple important permettant la rotation de la paroi tubulaire avec les frottements du tampon applicateur.

Selon un autre mode de réalisation, l'organe d'application est en partie au moins une bague annulaire montée autour de la surface de préhension, agencée pour se déplacer axialement le long de ladite surface entre une position de distribution dans laquelle l'organe d'application est en vis-à-vis du collecteur de distribution, ou communication fluidique avec celui-ci, pour le transfert de liquide nettoyant dans la réserve tampon et une configuration d'application pour l'application de liquide nettoyant sur la surface de préhension. De préférence, l'organe d'application comprend un support annulaire externe rigide à l'intérieur duquel est monté de manière coaxiale un tampon applicateur en forme d'anneau ayant une surface d'application en contact avec la surface de préhension. Dans ce mode de réalisation, le dispositif reste ergonomique et l'organe d'application du dispositif est moins encombrant puisqu'il peut être placé sur un côté de la poignée donc sans gêne lorsque celle-ci est opérée dans sa fonction de préhension.

Dans ce cas, la chambre de distribution de liquide de l'organe d'application comprend préférentiellement une série de sorties de liquide distribuées sur au moins une circonférence de la surface de préhension dans une zone de transfert ou de rechargement ; lesdites sorties étant agencées en regard d'une portion annulaire du tampon applicateur en position de rechargement ou d'alimentation distribution.

Dans le cas d'un déplacement axial de l'organe d'application vis-à-vis de la surface de préhension qui reste statique sur l'embase de la poignée, le dispositif est simplifié. Le tampon applicateur est séparable du collecteur de de distribution en liquide qui peut faire partie de la paroi de la surface de préhension et/ou de l'embase de la poignée; ce qui permet de simplifier l'organe d'application et d'éviter les problèmes de connexion fluidique associés au raccordement de pièces mobiles. Le collecteur de distribution statique fait ainsi office de station de recharge en liquide de position définie fixe pour le tampon applicateur ; lequel peut se mettre en position de repos et de rechargement ou d'alimentation en vis-à-vis du collecteur avant et/ou après un cycle de nettoyage.

En particulier, l'organe d'application du tampon applicateur peut être déplacer axialement le long de la surface de préhension au moyen d'un moyen d'entrainement à vis, à courroie, un engrenage sans contact du type électromécanique. A titre d'exemple, ce moyen d'entraînement comprend une vis sans fin s'étendant longitudinalement à l'intérieur de paroi tubulaire de la surface de préhension de la poignée et un écrou solidaire déplaçable le long de la vis sans fin et solidaire de l'organe d'application par une liaison traversant une ouverture ou une rainure longitudinale au travers de la paroi de la surface de préhension ou avec un couplage magnétique de l'anneau de l'organe d'application avec l'écrou de la vis sans fin (donc sans ouverture ou rainure nécessaire) permettant ainsi le déplacement axial de l'organe d'application lors de l'entraînement de la vis sans fin en rotation. Dans le cas d'un couplage magnétique, l'anneau de l'organe d'application peut être entrainé par un moteur linéaire capable de créer un champ électromagnétique de déplacement de l'organe le long de la surface de préhension.

Le contrôle des moyens d'entraînement et/ou des moyens d'alimentation peut se faire au moyen d'un moteur électrique et/ou de manière mécanique. L'énergie électrique et/ou mécanique peut avantageusement être une énergie collectée, au moins partiellement, par la force motrice appliquée par l'utilisateur sur la poignée lors de l'actionnement de la poignée sur un mécanisme d'ouverture et/ou de fermeture de l'ouvrant commandée par la poignée. Des modes préférentiels seront décrits plus loin.

Selon un mode de contrôle possible, le dispositif selon l'invention est commandé au moyen d'un contrôleur électronique telle qu'une unité centrale pouvant être logée dans la poignée ou en dehors comme dans un boitier, une partie de l'ouvrant ou un encadrement. Ainsi, l'unité d'actionnement comprend un contrôleur électronique configuré pour commander automatiquement l'action des moyens d'entraînement pour le déplacement relatif de la réserve tampon avec la surface de préhension et/ou commander la distribution en liquide par les moyens d'alimentation pour remplir en liquide la réserve tampon de l'organe d'application à partir du réservoir de liquide. Plus particulièrement, le contrôleur électronique commande l'actionnement d'un moteur électrique du moyen d'entraînement. Le contrôleur peut contrôler séquentiellement ou concomitamment l'alimentation en liquide par actionnement de la pompe et l'activation des moyens d'entraînement notamment le moteur électrique.

Selon un mode avantageux, le dispositif comprend au moins un capteur agencé pour détecter la présence du touché de la poignée par un utilisateur, ledit capteur étant associé au contrôleur électronique lequel est configuré pour actionner automatiquement les moyens d'alimentation du liquide et/ou d'entraînement, en réponse à une information de détection. La présence d'au moins un capteur permet ainsi d'activer la séquence de nettoyage selon un mode de détection pouvant être préétabli par programmation du contrôleur par exemple juste après la détection de la présence d'une main sur la poignée par le (les) capteur(s). Par exemple, après détection de présence par le (les) capteur(s), le contrôleur est programmé pour activer les moyens d'entraînement pour effectuer l'application du liquide sur la surface de préhension.

Le capteur peut être un capteur de présence ou d'effort à effet capacitif, optique, piézoélectrique ou piézo-résistif. Par exemple, un ou plusieurs capteurs sont positionnés sur ou à l'intérieur de la surface de préhension de la poignée notamment dans les zones les plus exposées comme au centre et/ou à une extrémité libre de la surface de préhension ou de l'embase.

Dans un mode d'alimentation possible utilisant une pompe électrique contrôlée, la pompe peut être activée suite à la détection par le/les capteur(s) pendant un temps prédéterminé correspondant au volume de remplissage de la réserve tampon. Le lancement de l'étape d'alimentation peut être éventuellement initiée à tout moment comme dès la détection du touché ou après la détection du touché et/ou après un temps prédéterminé. L'étape d'application ne peut être lancée quant à elle qu'après détection et une fois que le capteur ne détecte plus le touché ou la présence sur la surface ou l'embase.

Dans un autre mode de réalisation, le dispositif comprend une pompe actionnée par un moyen d'actionnement mécanique qui transmet un effort résultant d'un mouvement de pivot de la poignée actionnée par l'utilisateur, par exemple l'abaissement et/ou le retour horizontal de la poignée, comme lors de l'ouverture et/ou fermeture de l'ouvrant. La pompe peut être une pompe du type péristaltique ou à piston par exemple. Le moyen d'actionnement mécanique peut comprendre au moins une rampe ou un moyen de came lequel agit sur un moyen de compression péristaltique ou un piston de la pompe sous l'effet du mouvement de rotation de la poignée actionnée par la force motrice de l'utilisateur comme son abaissement et/ou sa relevée.

Selon un aspect avantageux de l'invention, le dispositif est configuré pour être autonome en énergie. De préférence, de l'énergie mécanique provenant de l'actionnement de la poignée est utilisée et stockée sous forme d'énergie électrique, mécanique ou une combinaison de ces deux types d'énergie pour être ensuite utilisée pour actionner la pompe et/ou les moyens d'actionnement pour le déplacement relatif de l'organe d'application et/ou de la surface de préhension.

Ainsi, le dispositif comprend au moins un bloc de stockage et d'inversion ou un organe de récupération d'énergie mécanique par des moyens de transmission de couple du fait de la rotation de la poignée sur un mécanisme d'ouverture et/ou fermeture d'un ouvrant (porte, fenêtre, etc.), pour le stockage de cette énergie mécanique et/ou de transformation et stockage en énergie électrique et enfin de redistribution de cette énergie mécanique ou électrique stockée à l'unité d'actionnement ou aux moyens d'entraînement ou à un ensemble formé d'un bloc de translation et un bloc de couplage qui commande le déplacement de la surface de préhension ou de l'organe d'application.

Selon un premier mode possible, l'organe de récupération d'énergie est du type électrique. En particulier, l'organe de récupération d'énergie peut comprendre un générateur de conversion d'énergie mécanique en énergie électrique tel qu'une dynamo, avec ou sans multiplicateur de vitesse, associée aux moyens de transmission de couple et comprend un accumulateur d'énergie électrique tel qu'au moins un supercondensateur et/ou une batterie configurée pour stocker l'énergie électrique ainsi générée par le générateur et la mettre à disposition pour alimenter les moyens d'entraînement et/ou le contrôleur électronique.

Selon un mode de réalisation préféré, l'organe de récupération et de restitution d'énergie est du type mécanique.

En particulier, l'organe de récupération est un organe d'accumulation d'énergie mécanique, de restitution et de transmission d'énergie mécanique ou bloc de stockage et d'inversion comprenant au moins un mécanisme de transmission avec au moins un arbre pour transmettre un couple à un mécanisme de fermeture d'ouvrant, au moins un premier ressort agencé pour emmagasiner l'énergie mécanique lors du déplacement dudit arbre, un premier embrayage unidirectionnel et un ensemble de loquets cycliques actionnés par un arbre à came pour restituer l'énergie emmagasinée par le premier ressort à des moyens de transmission et de couplage, ou bloc de translation et bloc de couplage, configurés pour déplacer l'organe d'application relativement à la surface de préhension dans une première direction.

Selon un exemple préférentiel, l'organe d'application est déplacé sur la surface de préhension depuis une position d'alimentation à une position de fin de course. Dans ce cas, l'organe d'application de préférence de forme annulaire se déplace de préférence axialement sur la surface de préhension comme précédemment mentionné. Selon un autre exemple, c'est la surface de préhension, par exemple de forme hélicoïdale (comme décrit dans un mode de la présente demande) qui est déplacée en rotation vis-à-vis d'un organe d'application en forme de barre nettoyante.

Selon un exemple, les moyens de transmission et de couplage peuvent comprendre un bloc de translation et un bloc de couplage pour déplacer l'organe d'application en translation sur la surface de préhension. Le bloc de translation peut comprendre un ensemble formé par une vis sans fin et un écrou par exemple ou un ensemble de courroie et poulies associé à un élément de connexion associé au bloc de couplage.

Le bloc de couplage peut former un couplage direct par tige ou équivalent avec l'organe d'application. Alternativement, le bloc de couplage peut être un organe de couplage magnétique permettant de transmettre l'effort (force ou couple) du bloc de translation à l'organe d'application sans liaison mécanique.

De plus, l'organe d'accumulation d'énergie mécanique, de restitution et de transmission d'énergie mécanique ou bloc de stockage et d'inversion peut comprendre des moyens d'inversion de mouvement configurés pour déplacer l'organe d'application relativement à la surface de préhension dans une seconde direction opposée à la première direction.

Selon un exemple préférentiel, ces moyens d'inversion comprennent un second embrayage cyclique et un second ressort configurés pour déplacer l'organe d'application dans la seconde direction, par exemple pour rappeler l'organe d'application de sa position de fin de course à sa position d'alimentation, ledit second embrayage étant arrangé entre le second ressort et le premier ressort afin d'entraîner librement l'organe d'application sans contrainte du premier ressort.

Selon un aspect de l'invention, les moyens d'alimentation en liquide comprennent au moins une pompe actionnée de manière contrôlée électroniquement par une unité électronique ou mécaniquement par des moyens d'actionnement mécanique lors de l'actionnement de la poignée de préférence le pivotement de celle-ci autour de son axe d'actionnement pour l'actionnement d'un mécanisme d'ouverture/fermeture de l'ouvrant. De préférence, la pompe est une pompe péristaltique ou une pompe à piston.

Selon un aspect possible de l'invention, le réservoir de liquide peut être contenu dans un conteneur amovible connectée par des moyens de connexion détachables à au moins une interface de connexion des moyens d'alimentation. L'interface de connexion peut être contenue dans le châssis de la poignée ou dans un boitier solidaire de l'ouvrant. L'interface peut être munie de moyens de connexions complémentaires et communiquant avec l'organe d'application via au moins un conduit.

D'autres caractéristiques et avantages de l'invention ressortiront à la lecture de la description qui suit, en référence aux figures annexées, qui illustrent :
- La figure 1 est une vue de face schématique générale du dispositif de nettoyage de la poignée installé sur une porte selon un premier mode de réalisation de l'invention ; de la figure 1 ;
- La figure 2 est une vue en perspective et par transparence d'une partie du dispositif, en particulier de la partie de préhension de la poignée selon le premier mode de réalisation ;
- La figure 3 est une vue de face de la poignée de la figure 2 ;
- La figure 4 est une vue en coupe selon un plan vertical médian de la partie de préhension de la poignée de la figure 3 ;
- La figure 5 est un ordinogramme d'un exemple de méthode de nettoyage de la poignée selon le mode du dispositif des figures 1 et 2 ;
- La figure 6 est une vue générale en perspective d'un dispositif de nettoyage de la poignée installé sur un ouvrant selon un second mode de réalisation de l'invention ;
- La figure 7 est une vue générale en perspective par transparence du dispositif de la figure 6 ;
- La figure 8 est une vue en perspective et par transparence d'une partie du dispositif, en particulier de la poignée selon le second mode de réalisation ;
- La figure 9 est une vue de face de la figure 8 ;
- La figure 9a est une vue en coupe transversale de la paroi tubulaire de la surface de préhension seule ;
- La figure 10 est une vue élargie d'un détail de la figure 9 ;
- La figure 11 est une illustration schématique d'un organe de récupération d'énergie électrique du dispositif ;
- La figure 12 est une vue en perspective et par transparence d'une partie de la poignée comprenant une partie de l'organe de récupération d'énergie de la figure 11 ;
- La figure 13 est une vue en perspective partielle montrant un détail de l'organe de récupération d'énergie de la figure 12, en particulier, les moyens de transmission du couple par la poignée à l'ouvrant ;
- La figure 14 est une vue en perspective partielle montre un autre détail l'organe de récupération d'énergie de la figure 12 en particulier la liaison cinématique entre le moteur et les moyens de transmission du couple ;
- La figure 15 montre un élément des moyens de transmission du couple du dispositif des figures 12 à 14 ;

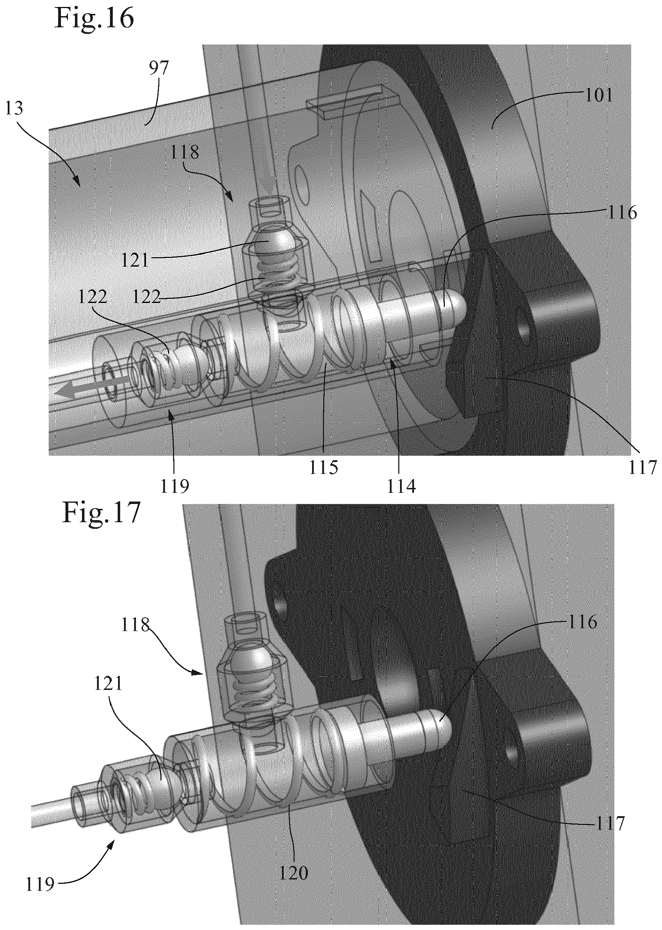

- La figure 16 montre un détail des moyens d'alimentation en liquide de la poignée en particulier une pompe à piston actionnées pas le mouvement de la poignée lors de l'actionnement d'un mécanisme d'ouverture et/ou fermeture de l'ouvrant ;
- La figure 17 montre un détail des moyens d'alimentation en liquide de la poignée sans le châssis de la poignée ;
- La figure 18 est une vue générale en perspective d'un dispositif de nettoyage de la poignée installé sur un ouvrant selon un second mode de réalisation de l'invention dans une configuration d'alimentation en liquide ou de repos;
- La figure 19 est une vue générale en perspective d'un dispositif de nettoyage de la poignée installé sur un ouvrant selon un second mode de réalisation de l'invention dans une configuration de nettoyage;
- La figure 20 est une vue en coupe selon un plan horizontal médian du dispositif de nettoyage selon le second mode de réalisation;
- La figure 21 est une vue en perspective sans son habillage externe complet en particulier le recouvrement de la poignée par la paroi tubulaire de préhension du second mode de réalisation;
- La figure 22 est une vue élargie d'un détail du dispositif de la figure 20 en particulier du mécanisme d'entraînement, de récupération et de restitution d'énergie du dispositif (« Organe d'entraînement et d'énergie »);
- La figure 23 est une autre vue en perspective externe du dispositif de la figure 20 montrant l'organe d'entraînement, de récupération et de restitution de l'énergie mécanique du dispositif ;
- La figure 24 est une vue selon un autre angle de de l'organe d'énergie et d'entrainement du dispositif de la figure 20 ;
- La figure 25 est une vue sans le bâti selon un autre angle de l'organe d'énergie et d'entraînement de la poignée ;
- La figure 26 est une vue élargie de détail de l'organe de la figure 24 ;
- La figure 27 est une vue élargie d'un détail de l'embrayage cyclique de l'organe de la figure 24 ;
- La figure 28 est une illustration schématique et cinématique de différents modes de réalisation possibles du dispositif en fonction de différents modules sélectionnés de l'organe d'accumulation et de restitution d'énergie mécanique et des moyens de transmission et de couplage.

### DESCRIPTION DÉTAILLÉE DE MODES DE RÉALISATION

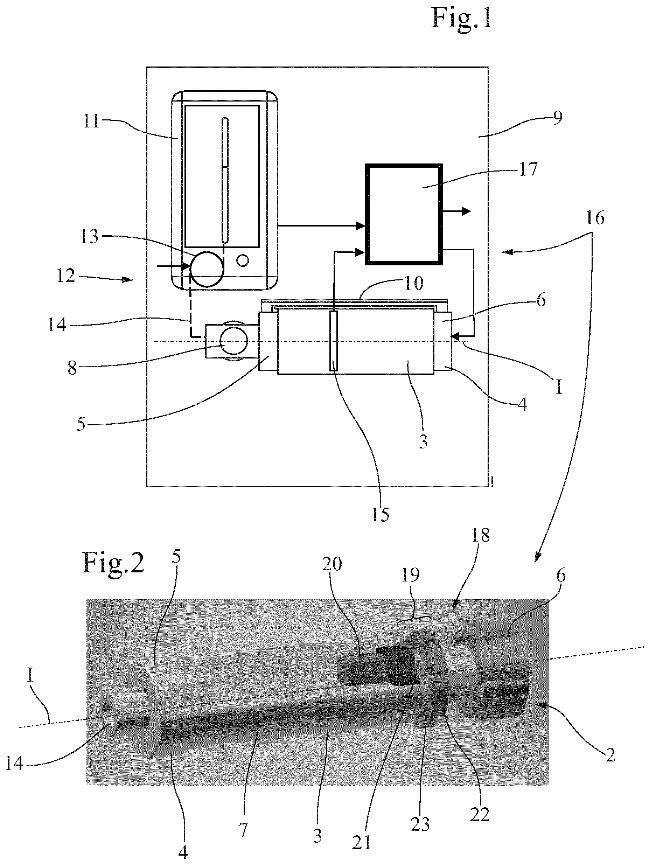

Les figures 1 à 4 illustrent un dispositif de nettoyage 1 selon un premier mode selon l'invention. Le dispositif comprend une poignée 2 avec une surface de préhension 3 qui peut former la surface d'une paroi de forme tubulaire ou partiellement tubulaire et qui s'étend le long d'un axe principal longitudinal médian l. La surface de préhension 3 peut être montée sur une embase 4 par exemple à deux portions d'extrémités 5, 6 reliées par un noyau central 7. L'une des extrémités 5 peut être connectée par un axe de pivot 8 perpendiculaire à l'embase et à la surface de préhension et se raccordant à un mécanisme d'ouverture (non visible) d'un ouvrant 9 telle qu'une porte. Une seconde poignée identique à la poignée 2 peut être prévue du côté opposé à l'ouvrant 9. Une seconde poignée standard peut être prévue de l'autre côté de l'ouvrant 9. Il est à noter que selon un mode possible, la poignée peut être montée de manière fixe, sans liaison avec un mécanisme d'ouverture, sur un ouvrant de type porte, fenêtre ou encore à un panneau, mur, sol ou tout autre support adéquat.

Le dispositif comprend aussi un organe d'application 10 configuré pour appliquer un liquide nettoyant sur la surface de préhension 3 et un réservoir 11 de liquide, contenant une solution de nettoyage, de capacité telle qu'elle permette une multitude de cycles de nettoyage. Des moyens d'alimentation 12 sont prévus pour alimenter l'organe d'application 10 en liquide en provenance du réservoir. Ces moyens 12 peuvent comprendre une pompe à liquide 13 ainsi qu'une conduite 14 reliant le réservoir à l'organe d'application 10.

Le dispositif peut comprendre au moins un capteur 15 sur la surface 3 pour la détection de la présence d'une main ou du touché sur la surface. Le capteur 15 peut être un capteur de présence par exemple de type capacitif ou un capteur optique tel qu'un capteur infrarouge. Le capteur peut être aussi un capteur de force ou de pression tel qu'une jauge de contrainte ou un capteur piézoélectrique. Le capteur peut aussi être un capteur de mouvement ou de force placé sur la poignée pour détecter un mouvement de la poignée sur son axe de pivot comme son abaissement lors de l'ouverture de l'ouvrant.

D'autres capteurs peuvent être prévus comme encore un capteur de niveau dans le réservoir.

D'une manière générale, l'organe d'application 10 est commandé par une unité d'actionnement 16 comprenant un contrôleur électronique 17 et des moyens d'entraînement 18 de l'organe d'application 10. Le contrôleur 17 peut être logé dans la poignée elle-même comme dans l'embase 4 ou en dehors comme dans un boîtier intégré ou associé au support 9. Le contrôleur peut être également présent dans le boitier du réservoir 11.

Un exemple est illustré à la figure 2. La surface de préhension 3 est montée sur l'embase pour pouvoir être entraînée en rotation par des moyens d'entraînement 18. La surface de préhension 3 peut être montée sur des roulements ou des surfaces de glissement (non illustrés). Le moyen d'entraînement 18 peut comprendre un engrenage 19 entraînée par un moteur électrique 20. L'engrenage 19 peut par exemple comprendre un pignon 21 relié au moteur et entraînant une bague annulaire 22 comprenant une denture sur sa surface interne. La bague 22 est montée solidaire de la surface de préhension 3 comme par des moyens d'arrêts 23 de type clavette et/ou par serrage par exemple. Ainsi la surface de préhension peut être entraînée en rotation angulaire autour de son axe longitudinal l par le moteur sur commande du contrôleur électronique 17. Un encodeur peut être prévu pour mesurer l'angle de rotation de la surface (non illustré) et permettre au contrôleur de contrôler l'amplitude de la rotation et notamment de stopper le moyen d'entraînement dès un seuil d'amplitude atteint.

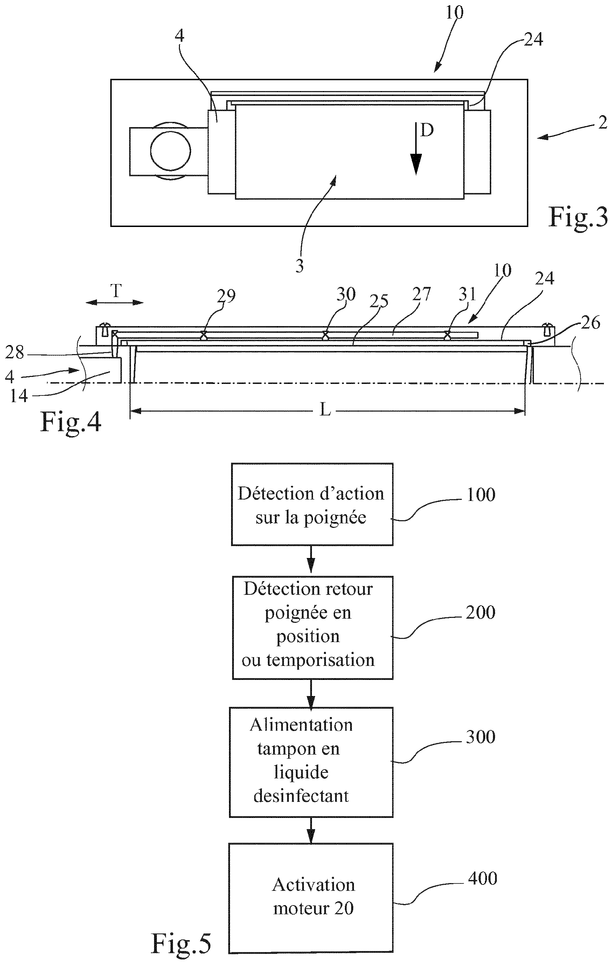

Un exemple d'organe d'application 10 est illustré aux figures 3 et 4 en détail. L'organe d'application 10 est fixe par rapport à l'embase et peut être relié par tout moyen de connexion approprié comme des vis par exemple. L'organe d'application à une forme de barre allongée dans la direction axiale de la surface de préhension qui comprend une réserve tampon 24 configurée pour contenir un volume de liquide nettoyant suffisant pour pouvoir appliquer une couche de liquide sur la surface de préhension lors d'un seul cycle d'application. De préférence, la réserve tampon comprend un tampon applicateur 25 ayant une structure permettant de stocker le liquide par effet de capillarité et/ou une structure poreuse ou d'interstices de rétention du liquide capable d'absorber le liquide, de faciliter son application et de réguler son étalement sur la surface 3. La structure peut être formée d'un matériau poreux, de préférence compressible, comme une éponge, une mousse ou un matelas de fibres ou peut être une structure de billes en polymère ou une structure en nid d'abeille par exemple. Le tampon applicateur 25 peut être contenu dans un logement 26 de l'organe d'application. Le tampon est dimensionné de largeur L en direction transversale T à la direction de déplacement de la surface de préhension ; sa largeur étant égale ou supérieure à la largeur de la surface de préhension de manière à assurer un recouvrement total de liquide sur celle-ci. L'organe d'application 10 comprend une chambre de distribution 27 reliée au conduit 14 des moyens d'alimentation par une entrée 28 laquelle s'étend de préférence sur toute la dimension transversale du tampon applicateur 25 et qui l'alimente en liquide par une pluralité de sorties de liquide 29, 30, 31 distribuées dans la direction transversale T. La disposition des sorties est telle que le tampon applicateur peut être rapidement et complètement chargé en liquide. L'alimentation du tampon peut se faire sur la face du tampon opposée à la face en contact avec la surface de préhension 3. Le liquide traverse ainsi facilement le matériau poreux du tampon pour pouvoir s'appliquer sur la surface 3.

Un exemple de fonctionnement du premier mode dispositif selon un mode possible peut être expliqué en relation avec la figure 5. Dans une première étape 100, une action sur la poignée est détectée par le ou les capteurs de présence 15. Cette information est traitée par le contrôleur électronique 17. Cette étape peut être suivie d'une seconde étape de détection de l'arrêt de l'action sur la poignée 200 ou la poignée à l'état de repos. Pour cela, le contrôleur 17 reçoit l'information du capteur sur l'absence de présence. Dans ce cas, le contrôleur initie l'étape suivante 300 consistant à alimenter la réserve tampon 24 en liquide nettoyant par activation de la pompe 13. Selon un mode alternatif possible, la seconde étape 200 consiste à appliquer une temporisation suivant l'étape de détection 100 avant d'initier l'étape suivante 300. Selon un mode alternatif possible, l'actionnement de la pompe et/ou de la rotation de la surface de préhension peut se faire par des moyens d'actionnement mécanique par l'action mécanique de déplacement de la poignée, par rotation ou autre mouvement, pour commander l'ouverture et/ou la fermeture de l'ouvrant. Une étape optionnelle de vérification du niveau de liquide peut être mise en oeuvre afin de vérifier la capacité du réservoir à remplir la réserve tampon. Si le niveau de liquide est insuffisant, la méthode peut être interrompue et un signal de rechargement émis par le contrôleur 17. Au cours de l'étape suivante 400, le contrôleur active les moyens d'entraînement 18, en particulier le moteur électrique 20, pour entraîner la surface de préhension en rotation angulaire selon une amplitude prédéterminée, par exemple de 360 degrés. La rotation de la surface a pour effet d'appliquer le liquide du tampon applicateur et de répartir son volume sur la surface de préhension. La couche de liquide peut être calibrée par un moyen de calibrage de préférence par une restriction comme une fente calibrée et/ou une lèvre transversale disposée entre la réserve tampon et la surface (non illustrée). Une fois la rotation achevée, le contrôleur 17 commande l'arrêt des moyens d'entraînement 18. L'angle de rotation peut être contrôlé par tout moyen comme par un encodeur, par une mesure électrique comme la consommation de puissance du moteur ou encore par une temporisation. Il faut noter aussi que l'étape d'alimentation peut être mise en oeuvre entre les deux premières étapes de détection 100, 200 ou après la dernière étape d'actionnement 400.

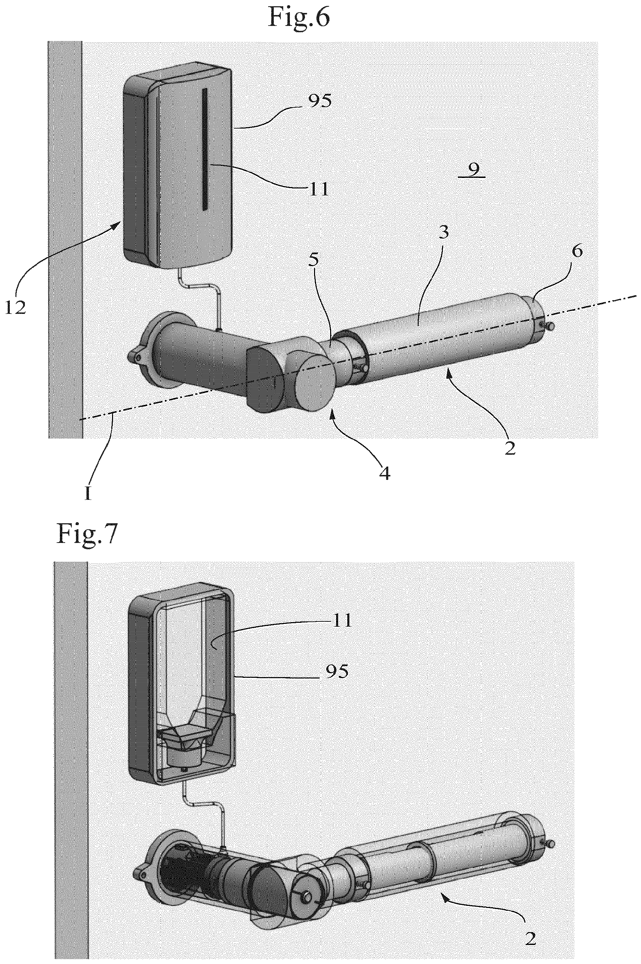

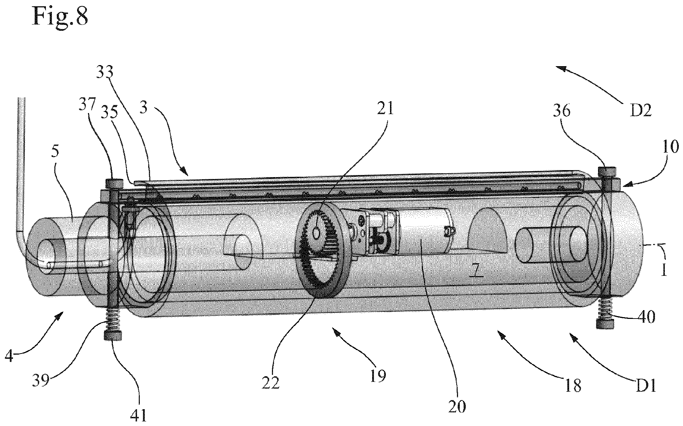

Un second mode de réalisation préféré de l'invention est illustré aux figures 6 à 10. Ce second mode est similaire au mode précédant et les mêmes moyens techniques portent les mêmes références dans les figures. Ainsi, le dispositif comprend les moyens d'alimentation 12 en liquide notamment un boitier 95 à l'intérieur duquel est logé un réservoir de liquide 11. Le dispositif comprend dans la partie de préhension de la poignée une embase 4 sur laquelle est montée en rotation selon l'axe longitudinal l une paroi tubulaire comprenant la surface de préhension 3. La paroi est entraînée en rotation selon l'axe longitudinal l par des moyens d'entraînement 18 logés dans l'embase comme sur un support ou noyau 7. Les moyens d'entraînement comprennent un engrenage 19 dont une bague 22 fixée intérieurement à la paroi tubulaire mobile, un pignon 21 relié à l'arbre d'un moteur électrique 20 d'entraînement de l'engrenage commandé par un contrôleur électronique (non représenté).

Une variante au mode précédant concerne la forme particulière de la surface de préhension 3 pour protéger l'organe d'application contre la contamination et le montage de l'organe d'application 10 sur l'embase de la poignée permettant de s'adapter à la forme particulière de la surface de préhension.

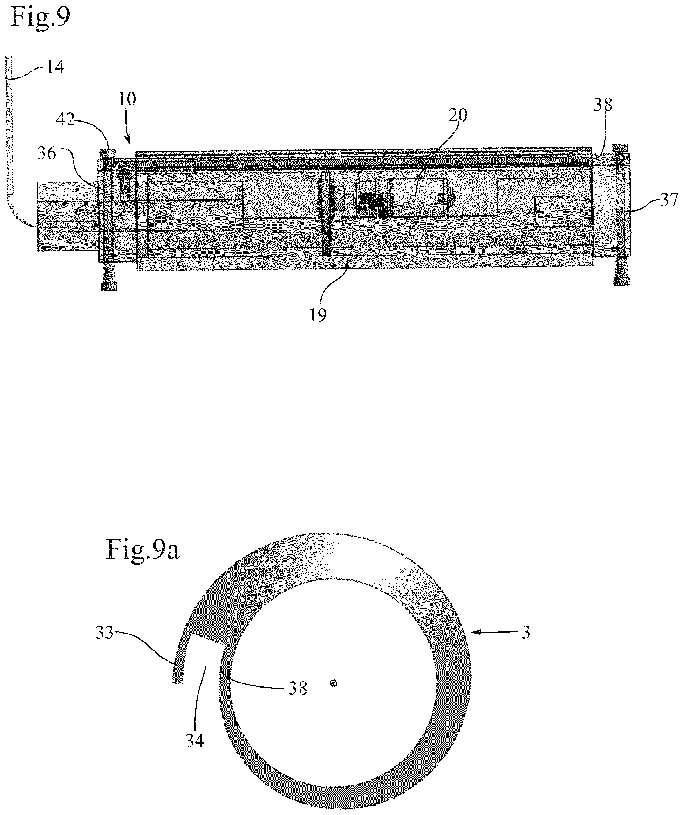

Ainsi, la surface de préhension possède une section transversale non-circulaire de préférence de forme hélicoïdale ou « d'escargot » qui comprend une extension de couverture 33 qui forme un logement 34 pour recevoir l'organe d'application 10. Le logement comprend une ouverture 35 qui s'étend en direction axiale pour permettre d'être traversé de chaque côté par l'organe d'application. L'ouverture s'étend aussi en direction circonférentielle pour permettre le passage de l'organe d'application 10 lors de la rotation de la surface de préhension. Ainsi, l'extension de couverture 33 de plus grand rayon vient en porte-à-faux par rapport à la base 38 de plus faible rayon de la surface de préhension comme le montre la figure 9a. Cette configuration permet de protéger l'organe d'application dans ce logement ouvert pour éviter tout contact avec la main de l'utilisateur dont la surface externe n'est pas nettoyée.

Dans ce montage, l'organe d'application 10 est monté en guidage radial sur l'embase 4 par des moyens de fixation 36, 37 sous forme de tiges de guidage traversant les extrémités 5, 6 de l'embase 4. Ces tiges de guidage sont plus longues que le diamètre de l'embase et sont montées en glissement dans l'embase de sorte que l'organe d'application 10 peut se déplacer radialement vis-à-vis de l'embase lorsqu'il est repoussé progressivement par la paroi de la surface de préhension 3 lors de la rotation de celle-ci. Un moyen de rappel élastique permet de maintenir l'organe en permanence contre la surface de préhension. Ce moyen de rappel peut être formé de ressorts 39, 40 montés sur la tige en appui sur un épaulement 41. D'autre part, la barre de l'organe d'application est maintenue par un épaulement 42 présent sur la tige du côté opposé à l'épaulement 41 sur lequel s'appuie le ressort. Les tiges vont donc se soulever radialement du côté de ce second épaulement 42 au fur et à mesure que l'épaisseur de la paroi de la surface de préhension augmente dans cette zone.

Le principe général de nettoyage du dispositif selon ce mode de réalisation est ainsi le suivant. L'unité d'actionnement 16 est configurée pour entraîner dans un premier temps la surface de préhension 3 dans une première rotation selon une première direction de rotation D1 depuis la position de repos de l'organe d'application 10 dans le logement 34 de l'extension de couverture 33. La distance angulaire est d'environ 360 degrés ou un peu plus en fonction de la longueur circonférentielle de l'extension de couverture. En tout cas, la rotation ne se termine que lorsque le tampon applicateur recouvre la surface externe de l'extension de recouvrement 33. A ce moment, l'unité d'actionnement stoppe la rotation de la surface de préhension et engage une rotation de celle-ci dans le sens opposé D2 jusqu'au retour de l'organe d'application 10 dans le logement 34. Cette seconde rotation est aussi d'environ 360 degrés d'angle. Durant ces mouvements de rotation, la barre de l'organe d'application translate radialement par le mouvement en guidage des tiges au travers de l'embase. Son retour contre la surface se fait par le rappel élastique des moyens de rappel élastiques 39, 40. Selon un autre principe de déplacement, l'organe d'application fait une première rotation de l'organe supérieure à 360° ; (l'organe de nettoyage faisant donc un « saut » de la zone haute à la zone basse de la surface de préhension) puis l'organe fait une seconde rotation en sens inverse jusqu'à son rangement dans le logement 34.

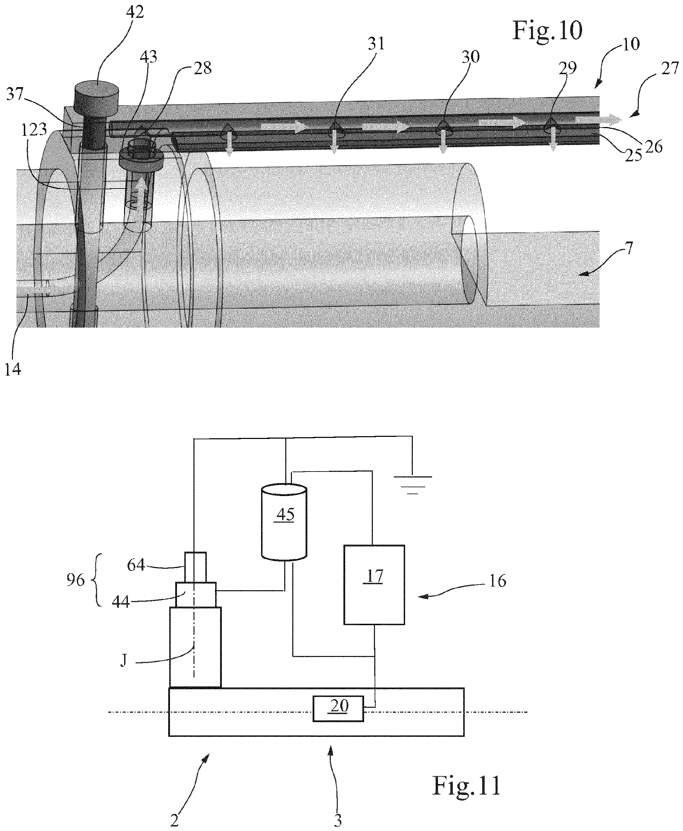

La figure 10 montre un détail de l'irrigation du tampon applicateur 25 de l'organe d'application 10. Une chambre de distribution 27 s'étendant de manière parallèle à l'axe longitudinal de la poignée est alimentée en liquide nettoyant au travers d'une entrée 28 radiale. Afin de permettre le déplacement radial de l'élément d'application sur la surface de préhension, un moyen d'extension 123 entre l'entrée 28 et le conduit 14 est prévu pour assurer la liaison fluidique lors du retour dans le logement de l'organe d'application après déplacement radial de l'organe d'application. Un joint d'étanchéité 43 peut être prévu entre l'embase 4 et le moyen d'extension 123. Le collecteur de distribution 27 comprend des sorties de liquide 29, 30, 31 distribuées longitudinalement ou axialement et s'étendant individuellement de manière radiale pour alimenter le tampon applicateur 25 en liquide. D'une manière préférentielle, l'alimentation en liquide se fait alors que l'organe d'application 10 est en position de repos dans le logement 34. Alternativement ou cumulativement, l'alimentation en liquide dans le tampon applicateur peut se faire pendant l'actionnement du mécanisme de nettoyage.

La figure 11 illustre un principe général de récupération d'énergie électrique par un organe de récupération d'énergie 96 pour alimenter les composants électriques de la poignée notamment l'unité d'actionnement 16. Ainsi, le dispositif comprend un générateur de conversion d'énergie mécanique en énergie électrique 44. Le générateur est relié à un mécanisme de transmission de couple 64 relié au mécanisme de fermeture de l'ouvrant lequel transmet au générateur l'énergie mécanique lorsque la poignée est actionnée manuellement en pivot autour d'un axe d'actionnement J. Le générateur 44 peut être un moteur, un motoréducteur ou une dynamo. Le dispositif comprend aussi au moins un accumulateur d'énergie électrique 45 reliée électriquement au générateur pour stocker l'énergie électrique ainsi produite. Un tel accumulateur peut un (super)condensateur et/ou une batterie rechargeable configurée pour stocker l'énergie électrique ainsi générée. L'accumulateur est relié électriquement à l'unité d'actionnement 16 pour alimenter les moyens d'entraînement et le contrôleur électronique 17.

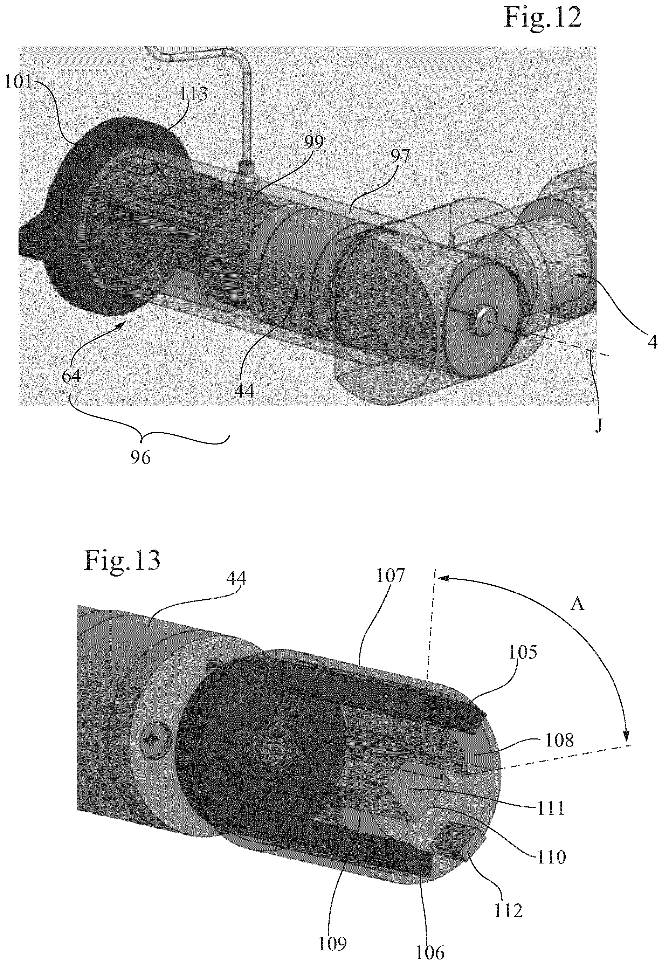

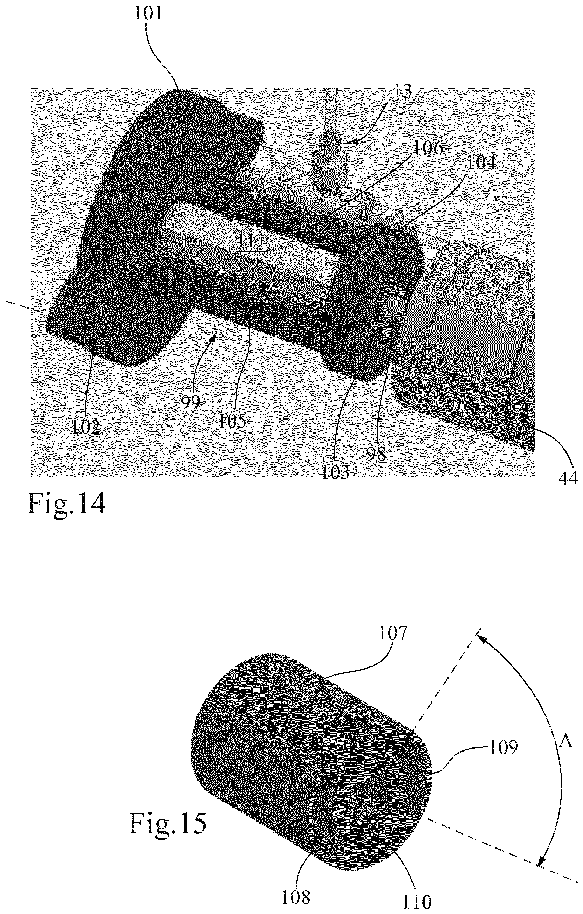

Les figures 12 à 15 illustrent un exemple préférentiel d'organe de récupération d'énergie 96 fonctionnant sur la base du principe général de la figure 11. Ainsi, l'organe comprend un générateur électrique 44, tel qu'une dynamo, logé à l'intérieur d'un châssis tubulaire 97 de la poignée lequel est connecté à l'embase de préhension 4 de manière rigide pour former un coude par exemple. Le moteur électrique 44 est inséré dans le châssis de manière fixe selon l'axe longitudinal J du châssis correspondant à l'axe de pivotement de la poignée et comprend un arbre de transmission 98 lequel est solidaire d'une embase 99 configurée pour être fixé sur une paroi de l'ouvrant (non représenté). L'embase peut être fixé à l'ouvrant plus particulièrement par un flasque 101 de l'embase 99. Le flasque 101 peut avoir la forme d'un disque ou autre muni de trous 102 permettant la fixation à l'ouvrant au moyen de vis, rivets ou autres. L'arbre 98 du moteur peut être connecté par exemple par une pièce de raccordement 103 à une extrémité proximale 104 de l'embase 99. L'embase comprend des barres de transmission, par exemple deux barres parallèles 105, 106, qui relient l'extrémité proximale 104 à l'extrémité distale formée par le flasque 101. L'organe de transmission du couple comprend de plus un cylindre 107 disposé entre les extrémités 101, 104 de l'embase 99, muni de fentes traversantes 108, 109 pour le passage glissant des barres de transmission 105, 106. Les fentes sont dimensionnées en forme d'ouvertures arquées dans la direction circonférentielle du cylindre et d'angle A correspondant à l'angle de déplacement en pivot de la poignée selon l'axe J lors du mouvement d'ouverture/fermeture du mécanisme de l'ouvrant. Par exemple, l'angle peut être de 25 à 60 degrés. Le cylindre 107 comprend un alésage traversant central 110 dans l'axe J de section transversale non-circulaire, par exemple, carrée afin de recevoir un longeron 111 de section complémentaire à la section de l'alésage, qui est destiné à traverser l'ouvrant pour transmettre le couple de l'actionnement manuel de la poignée sur un mécanisme d'ouverture/fermeture de l'ouvrant. Le cylindre comprend des moyens de blocage en rotation pour bloquer le cylindre vis-à-vis du châssis. Ces moyens de blocage peuvent comprendre une clavette 112 configurée pour s'engager dans un logement 113 du châssis de la poignée afin de solidariser le cylindre avec le châssis lors du pivotement de ce dernier lors de l'actionnement de la poignée.

Le fonctionnement de l'organe de récupération d'énergie 96 est le suivant. Lorsqu'un utilisateur abaisse la poignée, le châssis tubulaire 97 solidaire de l'embase de préhension 4 pivote autour de l'axe J d'un angle A, par exemple 30 degrés. L'arbre du moteur solidaire de l'embase 99 elle-même solidaire de l'ouvrant reste stationnaire alors que le moteur lui-même solidaire du châssis tourne avec le châssis de 30 degrés autour de l'axe J. Ce mouvement induit un courant électrique dans le moteur qui est collecté par l'accumulateur électrique. La répétition des cycles d'ouverture et/ou fermeture permet de charger l'accumulateur et d'alimenter le moteur électrique des moyens d'actionnement. Le couple de l'action mécanique sur la poignée est transmis par le châssis au cylindre au travers de la clavette 112 et du cylindre au longeron 111 au mécanisme de l'ouvrant. Les fentes 108, 109 permettent le mouvement de rotation sans déformation du cylindre par rapport à l'embase fixe. Il va de soi que d'autres mode d'exécution sont possibles comme une inversion mécanique à l'exemple illustré comme par exemple un générateur mobile en rotation dans le châssis et un arbre solidaire du châssis.

Les moyens d'alimentation en liquide du dispositif de nettoyage de l'invention selon ce mode peuvent aussi comprendre une pompe 13 solidaire du châssis 97 et actionnée par l'effet du pivotement du châssis 97 autour de l'axe J lors de l'actionnement de la poignée. Pour cela, la pompe 13 peut comprendre un piston 114 logé dans une chambre 115 de la pompe actionnée en déplacement axial par un doigt 116 agencé au contact d'un plan incliné ou rampe solidaire d'une partie fixe de la poignée, par exemple du flasque 101. La pompe comprend de préférence un moyen de retour élastique pour le piston comme un ressort 120 dans la chambre. La pompe peut comprendre une valve d'admission antiretour 118 en communication avec le réservoir de liquide au moyen d'une portion de conduit et une valve de refoulement antiretour 119 reliée à une portion de conduite alimentant l'organe d'application. Les valves peuvent être chacune une valve à bille 121 ou pointeau comprenant un moyen de rappel élastique tel qu'un ressort hélicoïdal 122 ou tout autre type de valve unidirectionnelle. Le plan incliné 117 est configuré par rapport à la pompe de manière à agir sur la position axiale du doigt et donc du piston dans la chambre. En position d'appui du doigt, le piston comprime le liquide dans la chambre et le liquide est refoulé en direction de l'organe d'application. La valve de refoulement 119 est alors comprimée en ouverture pour laisser passer le liquide. La valve d'admission montée en sens à l'inverse à la valve de refoulement est alors fermée par l'action de rappel du ressort sur la bille. En position de retour du doigt, le piston est en position de détente dans la chambre. La valve d'admission est ouverte par la différence de pression crée entre la chambre et la conduite d'entrée ; ce qui provoque l'aspiration du liquide dans la chambre et son remplissage. La valve de refoulement reste fermée par le rappel élastique du ressort 122 sur la bille 121.

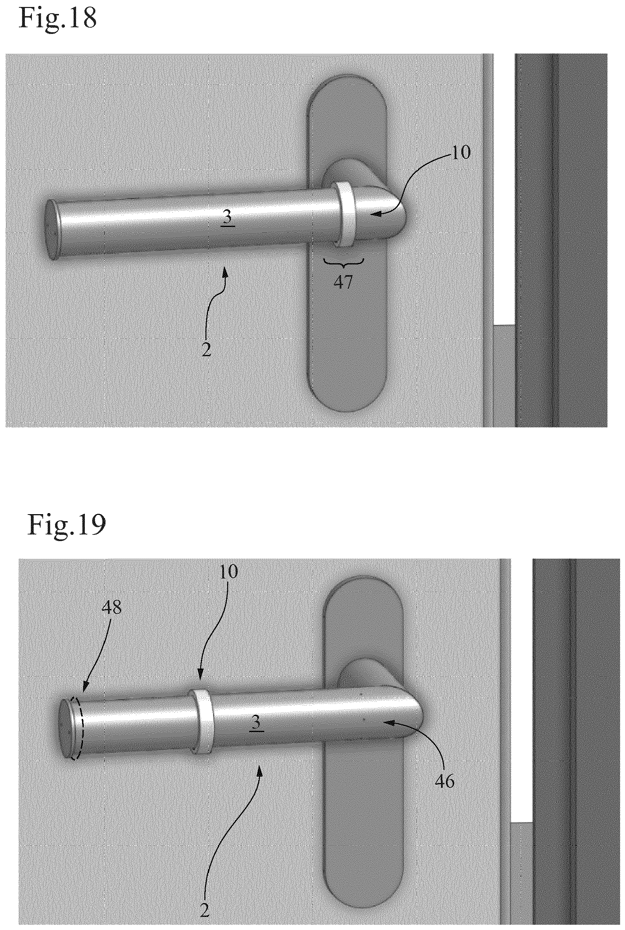

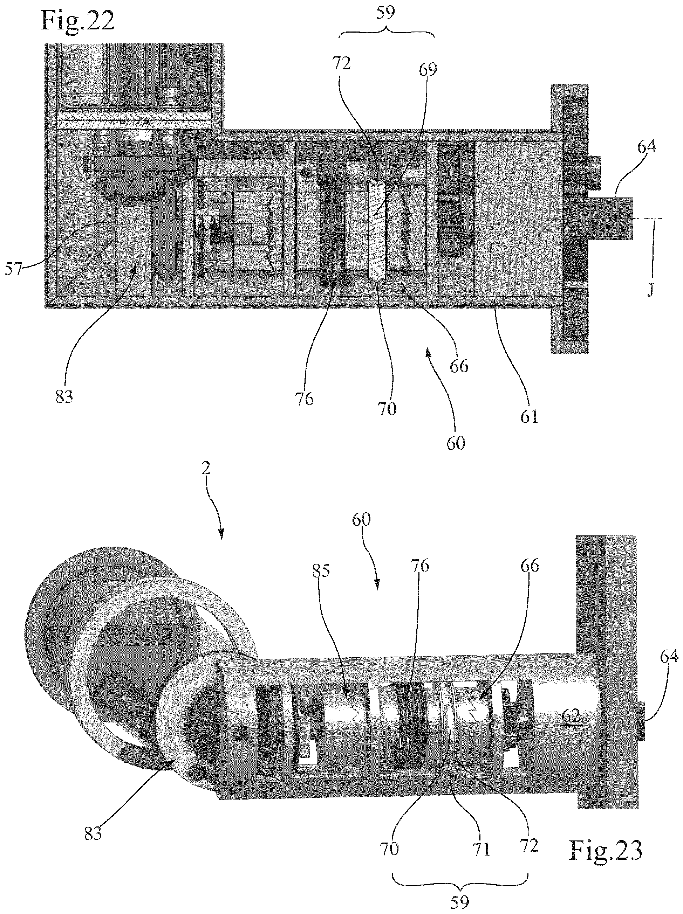

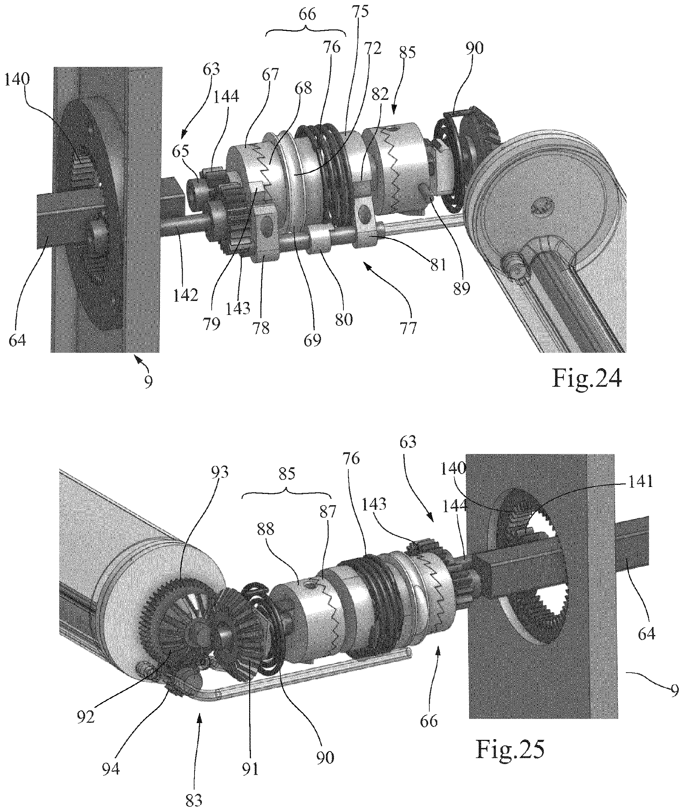

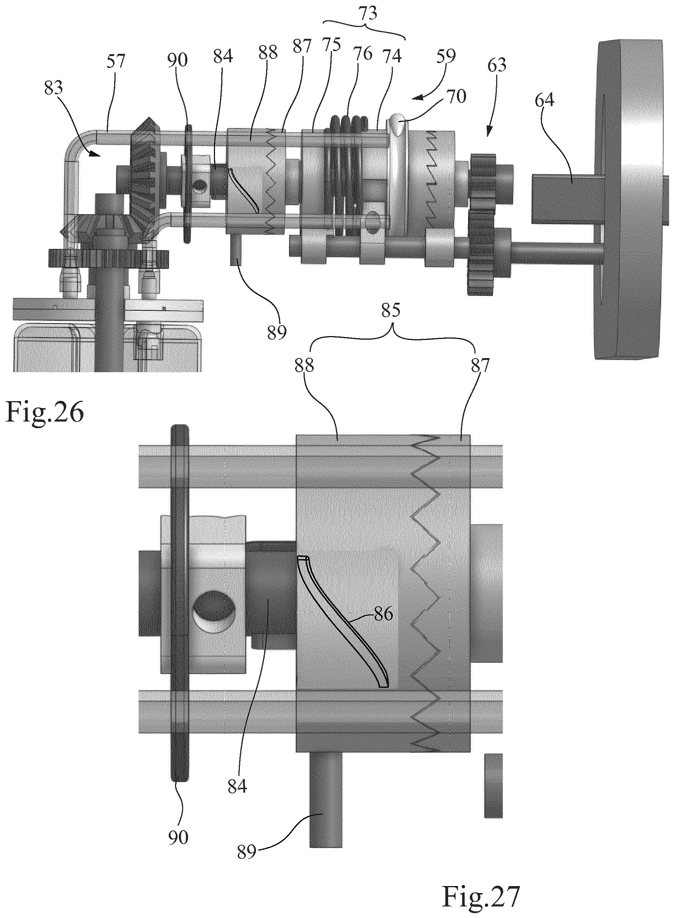

Un troisième mode de réalisation préféré du dispositif selon l'invention est illustré en relation avec les figures 18 à 27. Dans ce mode, la poignée 2 comprend une surface de préhension 3 statique et un organe d'application 10 qui se déplace longitudinalement ou axialement (c.-à-d. dans la direction d'axe l) le long de la surface lors du nettoyage. Notamment, l'organe d'application 10 a la forme d'une bague montée autour de la surface 3 depuis une position d'alimentation dans laquelle la bague recouvre une zone d'alimentation 46 de la poignée pour l'alimentation du liquide dans l'organe comme le montre la figure 18. L'organe d'alimentation est déplacé par des moyens d'actionnement qui seront détaillés plus loin le long de la surface de préhension 3 comme le montre la figure 19. Le déplacement peut se faire entre la position d'alimentation 47 (figure 18) et une position de fin de course 48 correspondant à l'extrémité de la surface de préhension (figure 19 : position identifiée en ligne pointillée). Une fois cette position de fin de course atteinte par l'organe d'application, ce dernier est rappelé en position d'alimentation pour être rechargé en liquide nettoyant par des moyens d'actionnement qui vont être décrits ci-après.

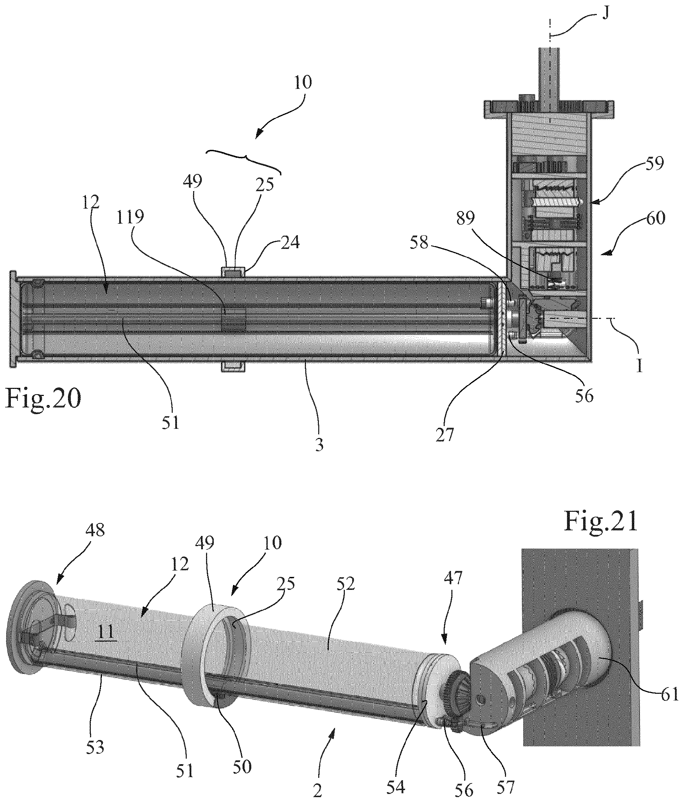

Comme le montrent les figures 20 et 21, l'organe d'application 10 comprend un tampon applicateur 25 disposé sur la surface interne d'un support annulaire rigide 49 de l'organe. Le tampon applicateur 25 est disposé de manière à rester en contact en permanence avec la surface de préhension 3 lors du déplacement axial de l'organe d'application. Le support annulaire 49 est relié à la poignée par une base 50 comprenant un écrou 119 lequel est monté et déplacé sur une vis sans fin 51 logée à l'intérieur de la paroi partiellement tubulaire 52 de la poignée et s'étendant au moins sur la largeur de la surface de préhension 3. La paroi 52 comprend un passage longitudinal traversant 53 s'étendant axialement pour permettre à la base du support annulaire de l'organe de se déplacer longitudinalement dans le passage entre la position d'alimentation 47 et la position de fin de course 48.

Dans la zone d'alimentation 47 est disposé un collecteur de distribution en liquide 27 configuré avec plusieurs sorties de liquide 54 distribuées radialement et préférablement sensiblement alignées dans un plan circonférentiel de la surface de préhension de manière à être recouvertes par le tampon applicateur 25 lorsque l'organe d'application est en position d'alimentation. Le collecteur de distribution 27 est de préférence alimenté par un connecteur 56 reliée à un conduit 57 lequel est relié à un réservoir de liquide 11 préférablement logé à l'intérieur de la poignée comme dans la paroi partiellement tubulaire 52 comprenant un connecteur de sortie 58 reliée au conduit 57. Le réservoir 11 peut être une cartouche amovible échangeable par exemple logée dans la poignée ou un réservoir fixe rechargeable. Une pompe 59 est disposée entre le réservoir et le collecteur pour alimenter l'organe d'application en liquide avant chaque cycle de nettoyage. La pompe peut être une pompe de type péristaltique qui comprime le conduit 57 souple et élastique. Toutefois, d'autres types de pompe peuvent être prévues comme une pompe à piston, à diaphragme ou autre.

Dans le mode de réalisation préféré, le dispositif est autonome en énergie pour l'alimentation du liquide par la pompe 59 et pour le déplacement de l'organe d'application lors du cycle de nettoyage. De plus, le dispositif est essentiellement mécanique de sorte qu'il peut être disposé contre l'ouvrant sans recours nécessaire à une alimentation électrique.

Pour cela, le dispositif comprend un organe d'accumulation d'énergie mécanique et de restitution et de transmission d'énergie mécanique 60 (appelé « organe d'énergie et d'entraînement » par la suite) à l'organe d'application dont le principe donné à titre d'exemple préférentiel est le suivant. L'organe d'énergie et d'entraînement 60 est ainsi configuré pour emmagasiner l'énergie mécanique d'actionnement de la poignée lors de son pivotement autour de l'axe J lors de l'abaissement de celle-ci pour l'ouverture de l'ouvrant. Ainsi, la poignée comprend un châssis tubulaire 61 de réception de l'organe d'énergie et d'entraînement 60 et de liaison cinématique entre le mécanisme de fermeture de la porte et l'organe d'application. Le châssis 61 de l'organe est sensiblement perpendiculaire à la paroi tubulaire 52 et à la paroi de la surface de préhension et comprend un bâti 62 logé à l'intérieur du châssis tubulaire sur lequel est monté en amont un mécanisme de multiplication de vitesse 63 relié au longeron 64 du mécanisme de fermeture de la porte. Le mécanisme de multiplication de vitesse 63 est configuré pour entraîner un arbre 65 de sortie du mécanisme sur un peu plus de 360 degrés lorsque le longeron 64 du mécanisme de fermeture de la porte est sollicité en pivotement selon un angle inférieur à 180 degrés (par exemple environ de 20° à 40°) correspondant à la course de l'abaissement de la poignée lors de l'ouverture de la porte. Le mécanisme de multiplication amont 63 est constitué d'un engrenage de roues et pignons dont la conception est à la portée de l'homme du métier. En particulier, le mécanisme 63 comprend une couronne dentée 140 formée dans la paroi de l'ouvrant 9 dans laquelle s'engrène un pignon satellite 141. Le pignon 141 est monté sur un arbre 142 qui porte un engrenage formé d'un premier pignon 143 qui entraîne un second pignon 144 monté sur l'arbre de sortie 65 du mécanisme. Le mécanisme 63 transmet ainsi un couple à un premier embrayage unidirectionnel ou antiretour 66 formé d'une partie amont crantée 67 et une partie aval crantée 68 capables de transmettre un couple dans la direction aval seulement. Les deux parties crantées 67, 68 coopèrent entre une position accouplée dans laquelle le couple est transmis de la partie crantée amont 67 à la partie crantée 68 et une position désaccouplée où aucun couple n'est transmissible en retour de la partie crantée aval 68 à la partie crantée amont 67. L'effet antiretour de l'embrayage est obtenu par un engagement asymétrique des dents des parties crantées 67, 68, configuré de telle manière à créer un accouplement mécanique que dans une seule direction en rotation et un désaccouplement dans la direction opposée. La partie aval 68 de l'embrayage est solidaire de la pompe péristaltique 59 qui comprend une partie de disque rotatif 69 entraînée par le couple transmis par l'embrayage avec une rainure annulaire 72 dans la partie de disque ainsi qu'un passage tubulaire rigide fixe 71 dans le bâti pour le passage du conduit flexible. La zone de passage comprend une came 70 qui comprime périodiquement le tube lors de chaque rotation de la partie rotative 69. La partie rotative 69 est elle-même solidaire d'un ensemble 73 de loquets cycliques comprenant un loquet amont 74 en forme de roue et un loquet aval 75 en forme de roue reliés par un premier ressort principal de torsion 76. Il faut toutefois noter que la rainure annulaire 72 est préférablement omise de façon à faciliter le couplage et découplage de l'embrayage à effet antiretour. L'activation des loquets est réglée par un arbre à came 77 disposé le long de l'organe. L'arbre à came 77 comprend une première dent 78 disposée sur l'arbre et à proximité de la partie amont 67 de l'embrayage pour engager un doigt 79 disposé sur la surface de de cette partie.

L'arbre à came 77 comprend aussi une deuxième dent 80 sur lequel peut prendre appui un ressort de torsion 76 pour éviter à celui-ci de se détendre lorsque la poignée revient en position de repos (de préférence horizontalement). Enfin l'arbre 77 comprend une troisième dent 81 qui s'engage cycliquement sur un doigt 82 du loquet aval 75 de manière à libérer le loquet lorsque la première dent 78 est en prise avec le doigt 79 et donc restituer l'énergie du ressort en aval sur une transmission 83 reliée à la vis sans fin qui déplace l'organe d'application. La transmission 83 est entraînée par un arbre 84 relié à l'ensemble 73 de loquets par l'intermédiaire d'un second embrayage cyclique bidirectionnel 85 constitué de deux parties crantées 87, 88. Ce second embrayage est configuré pour transmettre un couple à la transmission 83 en position accouplée pour le déplacement de l'organe d'application sur la surface de préhension, en aller dans la position de fin de course et en position désaccouplée lors du retour dans la position d'alimentation lors du nettoyage. Le passage à la position désaccouplée du second embrayage 85 est effectué au moyen d'un chemin de came 86 porté par la surface de la partie aval 88 et d'un doigt 89 supporté par le bâti de la poignée. L'organe d'énergie et d'entraînement comprend un second ressort de torsion 90 relié à l'arbre de transmission 84 et au bâti afin de procurer l'énergie nécessaire au retour de l'organe d'application de sa position de fin de course à sa portion d'alimentation. L'énergie de ce second ressort est emmagasinée lors de la phase aller de l'organe d'application et de restitution de l'énergie du premier ressort 76. Ce second ressort 90 se tend à mesure que le premier ressort 76 se détend. Le second ressort 90 est choisi avec une constante de raideur plus faible que le premier ressort 76 de façon à permettre l'emmagasinement d'une partie de l'énergie par le second ressort. Le second ressort pourrait être remplacé par tout autre type de ressort comme un ressort linéaire ayant une force de rappel constante ou proportionnelle fixé, par exemple directement à la bague de support 49. Les deux ressort 76 et 90 pourraient eux-mêmes être des ressorts à couple constant ou quasi-constant.

La transmission ou multiplicateur 83 en sortie de l'organe d'énergie et d'entraînement peut être réalisée par un engrenage d'au moins deux pignons 91, 92 arrangés à 90 degrés ou tout angle approprié et éventuellement un engrenage secondaire avec une roue 93 coaxiale au pignon de sortie 92 et engrainant un pignon 94 couplé à la vis sans fin. Toute autre transmission appropriée permettant l'entraînement et le réglage précis de la course de la bague entre les deux positions de course 46, 48 de la bague est possible. Un autre type de transmission peut être une transmission par courroie ou câble ou un couplage magnétique. Dans ce dernier cas, l'écrou d'entrainement 50 entraine un aimant qui lui entraine la bague de l'organe de nettoyage, celle-ci comprenant un corps aimanté ou ferromagnétique par exemple logé à l'intérieur de la bague. Afin d'éviter les phénomènes d'arcboutement, la bague peut être coupée à une multitude d'aimants répartis de manière homogène dans la bague ainsi que dans le périmètre interne de la surface de préhension translaté par un ou plusieurs actuateurs linéaires (vis - écrou ou courroie).

Le fonctionnement général de l'organe d'énergie et d'entraînement est le suivant. Lors de l'abaissement de la poignée 2, le premier embrayage unidirectionnel 66 est en position d'accouplement. Le loquet amont 74 est en position libre alors que le loquet aval 75 est en position bloquée. L'ensemble de loquets cyclique 73 isole ainsi le ressort de torsion principal 76 qui se tend et emmagasine l'énergie mécanique du mouvement d'abaissement de la poignée. Le second embrayage bidirectionnel 88 est de préférence en position accouplée. Lors de cette phase, l'organe d'application 10 est en position d'alimentation en vis-à-vis du collecteur de distribution de liquide. Lors de cette phase de rotation sur un tour environ, la pompe péristaltique 59 est aussi actionnée en rotation ce qui engage la came 70 sur le conduit de sorte que le liquide est pompé du réservoir vers le tampon applicateur remplissant ainsi celui-ci en liquide de nettoyage.

Lorsque la poignée est désengagée et remonte de sa position abaissée, le premier embrayage antiretour 66 se désaccouple et l'ensemble de loquets est bloqué par le mécanisme 81, 82 de l'arbre à came. Arrivé à la position de déplacement finale de la poignée, le doigts 79 vient pousser les dents 78 et 81, libérant ainsi le doigt 82 et le premier ressort de torsion 76. Le premier ressort de torsion 76 est alors libéré et le doigt 82 de la roue aval 75 du loquet est libérée ce qui permet la transmission du couple emmagasiné par la détente du ressort à la transmission ou multiplicateur aval 83. L'organe d'application est par conséquent déplacé de la position d'alimentation à la position de fin de course permettant ainsi le nettoyage de la poignée. Lors de cette phase, l'embrayage est en position accouplé et le premier ressort 76 tend le second ressort 90 qui emmagasine donc de l'énergie dans cette phase aller de l'organe 10.

Un fois l'organe d'application 10 arrivé en position de fin de course, le second ressort est tendu à sa tension maximale. La roue aval 75 du loquet cyclique 73 n'est alors pas encore en position bloquée. Le second embrayage cyclique 66 se désaccouple à la fin de cette première rotation, la roue avale 75 n'est pas encore bloquée. Le second embrayage cyclique 85 se désaccouple alors (le doigt 89 engagé dans le chemin de came 86 sépare éloigne les parties crantées 87, 88 de l'embrayage l'une par rapport à l'autre). Le second ressort 90 se détend ce qui entraîne la transmission en sens opposé et entraine l'organe d'application 10 qui retourne en position d'alimentation.

Une fois l'organe en position d'alimentation, la poignée est au repos et un nouveau cycle d'alimentation et de nettoyage peut recommencer lorsque la poignée est à nouveau abaissée par un utilisateur.

Il faut noter que le dispositif peut fonctionner avec deux poignées opposées et disposées de part et d'autre de l'ouvrant. Le dispositif est parfaitement autonome en énergie et cette énergie est renouvelable puisqu'elle n'a pour origine que la force musculaire.

Il faut noter que l'organe d'énergie et d'actionnement du mode des figures 18 à 27 pourrait être toutefois remplacé une unité d'actionnement comprenant un contrôleur électronique, un moteur électrique d'actionnement ou en utilisant un moteur linéaire, optionnellement un ou plusieurs capteurs de position de la bague annulaire, et optionnellement aussi un organe de récupération d'énergie de la poignée, de transformation et de stockage en énergie électrique pour l'alimentation des éléments électriques et électroniques du dispositif.

De même le mode des figures 6 à 10 peut aussi être accouplé à un organe d'énergie et d'entraînement mécanique.

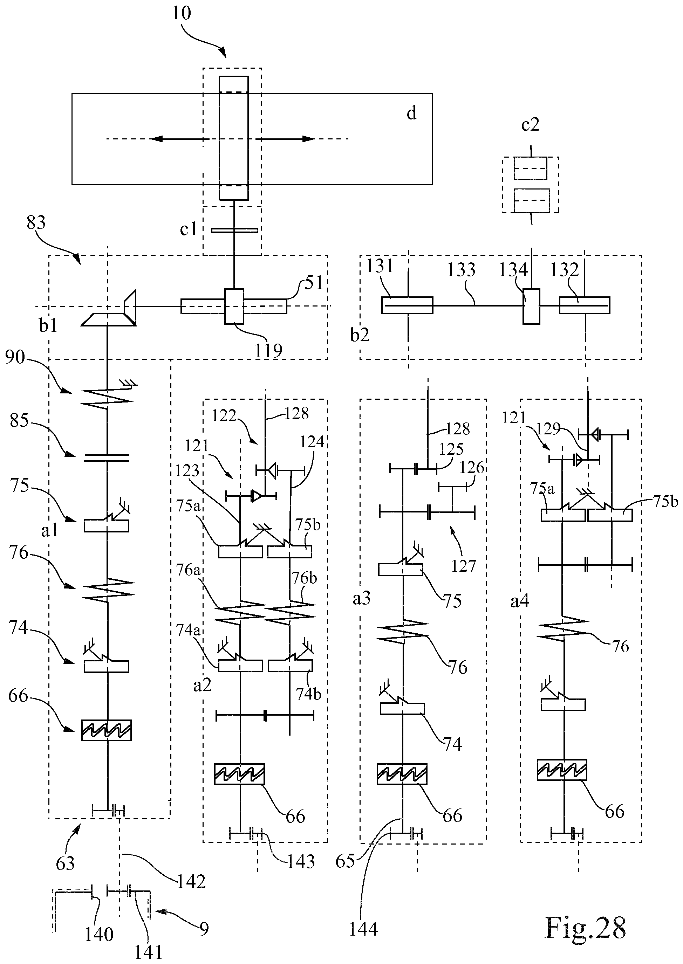

La figure 28 représente plusieurs combinaisons possibles de modules du dispositif avec stockage et restitution d'énergie mécanique.

D'une manière générale, le dispositif comprend un organe d'accumulation et de restitution d'énergie mécanique sous forme d'un bloc de stockage et d'inversion a1, a2, a3 ou a4. Le bloc de stockage et d'inversion est relié à un bloc de translation b1 ou b2 et à un bloc de couplage c1 ou c2 pouvant être relié à l'organe d'application 10 ou la surface de préhension 3. Les blocs de stockage et d'inversion a1-a4, de translation b1-b2 et de couplage c1-c2 peuvent être sélectionnés selon différentes combinaisons possibles.

Selon une première configuration du dispositif, le bloc de stockage et d'inversion a1, le bloc de translation b1 et le bloc de couplage c1 font partie du dispositif décrit dans le mode des figures 18 à 27. Les mêmes références sont utilisées pour désigner les mêmes éléments.

D'autres modes de réalisation sont envisageables, les blocs de stockage et d'inversion a1, a2, a3 et a4 étant interchangeables de même que les blocs de translation b1 et b2 ainsi que les blocs de couplage c1 et c2.

A titre d'exemple une combinaisons a2 - b1 - c2 est une solution possible. Les réducteurs et multiplicateurs de vitesse ainsi que les mécanismes d'ouverture des dents des loquets ne sont pas représentés dans cette figure. Chaque élément est représenté de manière schématique, sans ancrage à une référence et sans souci d'échelle.

Le bloc de stockage et d'inversion a2 est constitué (par ordre d'actionnement) d'un embrayage anti retour couplé en sortie au premier ressort 76a qui est lui-même bloqué par un système de loquets/dents 75a en entrée et en sortie de celui-ci, tout comme pour le bloc a1. Ce système permet au ressort d'être armé lors de la descente de la poignée sans être désarmé lors de la remontée de celle-ci. Parallèlement, un second système de ressort bloqué 76b, entre deux loquets/dents 74b, 75b est lui aussi armé lorsque l'utilisateur actionne la poignée en pivotement (par exemple pour la descendre lors de l'ouverture/fermeture de l'ouvrant). Deux engrenages unidirectionnels 121, 122 (couplés dans un sens de rotation, en roue libre dans l'autre sens de rotation) sont disposés en sortie d'arbres de manière à ce que le premier arbre 123 entraine l'anneau dans sa phase allé tandis que le second arbre 124 entraine l'anneau dans sa phase de retour, sans couplage de l'arbre 124 lors de la phase allée et sans couplage de l'arbre 123 dans la phase retour.

Lorsque la dent du loquet 75a de sortie du premier ressort 76a est ouverte, ce dernier se décharge entrainant l'anneau de l'organe d'application 10 sur sa phase aller. Une fois arrivé en bout de course, la dent du loquet de sortie 75b du second ressort 76b est à son tour ouverte, entrainant l'anneau dans sa phase de retour. Les dents des loquets sont cycliquement ouvertes par un système d'arbre à cames mû par l'arbre central (non illustré). Ils sont généralement passivement fermés par des ressorts de rappels immédiatement après avoir été ouverts.

Le bloc de stockage et d'inversion a3 est très similaire au bloc a1, à l'exception près que l'inversion du sens de rotation se fait avec un moyen d'inversion similaire à un système de boite à vitesse. L'anneau de l'organe d'application passe de la phase aller à la phase retour lorsque le pignon 125 lié à l'arbre de sortie 128 est déplacé relativement en translation pour venir se coupler au pignon 126 du deuxième arbre 127, qui lui tourne dans la direction opposée.

Dans ce cas le ressort doit assurer l'aller et le retour de l'anneau lors de sa détente.

Le bloc de stockage et d'inversion a4 est une combinaison des blocs a2 et a3. L'unique ressort 76 sert ici à mouvoir l'anneau de l'organe d'application dans sa phase aller et retour tout comme pour le bloc a3. Toutefois, le moyen d'inversion du sens de rotation fait par translation du pignon de sortie 129 pouvant être plus complexe qu'un double système d'anti retour, cette dernière solution est appliquée ici tout comme dans le bloc a2.

Le bloc de translation b2 représente une translation de l'anneau nettoyant faite par un système poulies 131, 132 et courroie 133.

Le bloc de couplage c2 représente un couplage magnétique. L'écrou 119 de la vis sans fin ou de l'élément de connexion 134 est solidaire d'un aimant ; tous deux se translatant dans un système clos. L'anneau dans lequel est inséré un système d'aimants ou de corps ferreux, est alors couplé magnétiquement à l'aimant de l'écrou 119 ou de l'élément 134. Plusieurs blocs d'aimants c2 et de translation (b1 ou b2) peuvent être nécessaires pour que l'anneau soit correctement guidé.

De nombreuses autres versions peuvent être imaginées stockant par exemple l'énergie lors de la descente et de la remontée de la poignée, composés aussi de systèmes d'anti retour, d'inverseurs, et de loquets à dents similaires à ceux présentés dans les blocs a1, a2, a3 et a4.

## Revendications

1. Dispositif de nettoyage d'une poignée (1) comprenant
- au moins une poignée (2) comprenant une surface de préhension (3) à nettoyer, de préférence faisant partie d'une paroi de forme tubulaire ou partiellement tubulaire,
- un organe d'application (10) pour appliquer un liquide nettoyant sur la surface de préhension (3),
- des moyens d'alimentation (12) en liquide nettoyant comprenant au moins un réservoir (11) pour recevoir et distribuer du liquide nettoyant à l'organe d'application,
**caractérisé en ce que** l'organe d'application (10) comprend au moins une réserve tampon (24) agencée au contact de la surface de préhension (3) et **en ce que** le dispositif comprend une unité d'actionnement (16) de l'organe d'application comprenant des moyens d'entraînement (51, 60 ; 18) configurés pour déplacer l'organe d'application (10) relativement à la surface de préhension (3) et/ou pour déplacer la surface de préhension (3) relativement à l'organe d'application (10), de façon à appliquer du liquide nettoyant depuis la réserve tampon (24) sur la surface de préhension lors de ce déplacement relatif.

2. Dispositif selon la revendication 1, **caractérisé en ce que** la réserve tampon (24) comprend un tampon applicateur (25) en contact avec la surface de préhension (3) ayant une structure permettant de stocker le liquide dans une structure poreuse ou d'interstices de rétention du liquide.

3. Dispositif selon la revendication 2, **caractérisé en ce qu'**il comprend un collecteur de distribution (27) de liquide nettoyant comprenant au moins une entrée de liquide (28) en communication avec le réservoir de liquide et une pluralité de sorties (29, 30, 31) de liquide distribuées en regard du tampon applicateur (24).

4. Dispositif selon les revendications 2 ou 3, **caractérisé en ce que** la poignée (2) comprend une embase fixe (4) sur laquelle est fixé l'organe d'application (10) et sur laquelle la paroi de la surface de préhension (3) est montée mobile selon au moins une direction en rotation autour de l'axe longitudinale (l) de la poignée ; les moyens d'entraînement (18) étant arrangés pour entraîner la paroi tubulaire (3) en rotation contre l'organe d'application (10) pour effectuer l'application d'une couche de liquide nettoyant sur la surface de préhension lors du déplacement selon ladite direction en rotation de la surface de préhension (3).

5. Dispositif selon la revendication 4, **caractérisé en ce que** la surface de préhension (3) de la paroi tubulaire mobile a une forme transversale sensiblement hélicoïdale et comprend une extension de couverture en porte-à-faux (33) configurée pour recouvrir l'organe d'application (10) en position de repos du dispositif ; ladite extension de couverture formant un logement (34) pour ledit organe (10) muni d'une ouverture (35) s'étendant à la fois axialement et circonférentiellement pour permettre le passage de l'organe d'application (10) lors du déplacement en rotation de ladite surface de préhension (3).

6. Dispositif selon la revendication 5, **caractérisé en ce que** l'unité d'actionnement est configurée pour entraîner la surface de préhension (3) selon une première rotation depuis le logement (34) en direction de ladite extension de couverture (33) pour effectuer l'application de liquide sur la surface de préhension, puis selon une seconde rotation dans la direction opposée jusqu'au rangement de l'organe d'application dans le logement (34).

7. Dispositif selon la revendication 5 ou 6, **caractérisé en ce que** l'organe d'application (10) est monté sur l'embase (4) de manière à pouvoir se déplacer radialement ou diamétralement vis-à-vis de la surface de préhension (3) à l'encontre d'au moins un moyen de retour élastique (39, 40) lors de la rotation de ladite surface de préhension (3).

8. Dispositif selon la revendication 3, **caractérisé en ce que** l'organe d'application (10) est formé en partie au moins d'une bague annulaire montée autour de la surface de préhension (3), agencée pour se déplacer axialement le long de ladite surface (3) entre une position de distribution dans laquelle il est en vis-à-vis du collecteur de distribution (27) pour le transfert de liquide nettoyant dans la réserve tampon (24) et une configuration d'application pour l'application de liquide nettoyant sur la surface de préhension (3).

9. Dispositif selon l'une quelconque des revendications précédentes, **caractérisé en ce que** l'unité d'actionnement (16) comprend un contrôleur électronique (17) configuré pour commander automatiquement l'action des moyens d'entraînement (18, 32) pour le déplacement relatif de la réserve tampon (24) avec la surface de préhension (3) et/ou commander la distribution en liquide par les moyens d'alimentation (12) pour remplir en liquide la réserve tampon de l'organe d'application à partir du réservoir de liquide (12).

10. Dispositif selon la revendication 9, **caractérisé en ce qu'**il comprend au moins un capteur (15) agencé pour détecter la présence du touché de la poignée par un utilisateur, ledit capteur étant associé au contrôleur électronique (17) lequel est configuré pour actionner automatiquement les moyens d'alimentation (12) du liquide et/ou d'entraînement (18, 32), en réponse à une information de détection.

11. Dispositif selon l'une quelconque des revendications précédentes, **caractérisé en ce qu'**il comprend au moins un bloc de stockage et d'inversion (a1-a4) ou organe de récupération d'énergie mécanique (96, 60) par des moyens de transmission de couple (63, 104, 140-144) du fait de la rotation de la poignée sur un mécanisme d'ouverture et/ou fermeture d'un ouvrant (9), pour le stockage de cette énergie mécanique et/ou de transformation et stockage en énergie électrique (45) et enfin de redistribution de cette énergie mécanique et/ou électrique stockée à l'unité d'actionnement (16) ou aux moyens d'entraînement (18, 32 ; 51, 60) ou à un ensemble formé d'un bloc de translation (b1, b2) et un bloc de couplage (c1, c2) qui commandent le déplacement de la surface de préhension (3) ou de l'organe d'application (10).

12. Dispositif selon la revendication 11, **caractérisé en ce que** l'organe de récupération d'énergie (96) comprend un générateur de conversion d'énergie mécanique en énergie électrique (44) tel qu'un générateur électrique tel qu'une dynamo, avec ou sans multiplicateur, associé aux moyens de transmission de couple et comprend un accumulateur d'énergie électrique (45) tel qu'au moins un (super)condensateur et/ou une batterie configurée pour stocker l'énergie électrique ainsi générée par le générateur (44) et la mettre à disposition pour alimenter les moyens d'entraînement (18 ; 51, 60) et/ou le contrôleur électronique (17).

13. Dispositif selon la revendication 11, **caractérisée en ce que** l'organe de récupération d'énergie (96) comprend un organe d'accumulation d'énergie mécanique, de restitution et de transmission d'énergie mécanique (60), ou bloc de stockage et d'inversion (a1-a4), comprenant au moins un mécanisme de transmission (63) avec au moins un arbre (64) pour transmettre un couple à un mécanisme de fermeture de l'ouvrant, au moins un premier ressort (76, 76a) agencé pour emmagasiner l'énergie mécanique lors du déplacement dudit mécanisme de transmission (63), un premier embrayage antiretour (66) et un ensemble de loquets cyclique (73) actionnés par un arbre à came (77) pour restituer l'énergie emmagasinée par le premier ressort (76) à des moyens de transmission et de couplage ou bloc de translation (b1, b2) et bloc de couplage (c1, c2) configurés pour déplacer l'organe d'application (10) relativement à la surface de préhension dans une première direction.

14. Dispositif selon la revendication 13, **caractérisée en ce que** l'organe d'accumulation d'énergie mécanique, de restitution et de transmission d'énergie mécanique (60) ou bloc de stockage et d'inversion (a1-a4) comprend des moyens d'inversion de mouvement (85,90, 122, 124, 125, 127, 129) configurés pour déplacer l'organe d'application (10) relativement à la surface de préhension dans une seconde direction opposée à la première direction.

15. Dispositif selon l'une quelconque des revendications précédentes, **caractérisée en ce que** les moyens d'alimentation en liquide (12) comprennent au moins une pompe (13) actionnée de manière contrôlée électroniquement par une unité électronique (17) ou mécaniquement par des moyens d'actionnement mécanique (63, 70, 71, 72) lors de l'actionnement de la poignée de préférence le pivotement de celle-ci autour de son axe d'actionnement (J) pour l'actionnement d'un mécanisme d'ouverture/fermeture de l'ouvrant; ladite pompe étant de préférence une pompe péristaltique ou une pompe à piston.
